# EUROPEAN PATENT SPECIFICATION

(11) **EP 1 899 452 B1**
(45) Date of publication and mention of the grant of the patent: **20.10.2010**
(21) Application number: 06757396.4
(22) Date of filing: 12.06.2006
(51) Int. Cl.: C12N 1/00

(54) **A METHOD FOR PRODUCING AN L-AMINO ACID USING A BACTERIUM OF THE ENTEROBACTERIACEAE FAMILY WITH ENHANCED EXPRESSION OF THE FUCPIKUR OPERON**
VERFAHREN ZUR HERSTELLUNG EINER L-AMINOSÄURE UNTER VERWENDUNG EINES BAKTERIUMS DER FAMILIE ENTEROBACTERIACEAE MIT VERSTÄRKTER EXPRESSION DES FUCPIKUR-OPERONS
PROCEDE DE PRODUCTION D'UN ACIDE L AMINE AU MOYEN D'UNE BACTERIE DE LA FAMILLE DES ENTEROBACTERIACEAE A EXPRESSION RENFORCEE DE L' OPERON FUCPIKUR

(30) Priority: 17.06.2005 RU 2005118796; 21.12.2005 US 743061 P
(43) Date of publication of application: 19.03.2008
(73) Proprietor: Ajinomoto Co., Inc., Tokyo 104-8315 (JP)
(72) Inventor: RYBAK, Konstantin, Vyacheslavovich, 117149 (RU); SLIVINSKAYA, Ekaterina, Aleksandrovna, 103055 (RU); SHEREMET'EVA, Marina, Evgenievna, 109202 (RU); SKOROKHODOVA, Aleksandra, Yurievna, 115304 (RU); KOZLOV, Yury, Ivanovich, deceased (RU)
(74) Representative: HOFFMANN EITLE
(86) International application number: PCT/JP2006/312195
(87) International publication number: WO 2006/135075

(56) References cited:
- WO-A-2004/090125
- CHEN ET AL: "Constitutive activation of L-fucose genes by an unlinked mutation in Escherichia coli" JOURNAL OF BACTERIOLOGY, vol. 159, no. 2, August 1984 (1984-08), pages 725-729, XP002394188
- CHEN ET AL.,: "The organization of the fuc regulon specifying L-fucose dissimilation in Escherichia coli K12 as determined by gene cloning." MOL GEN GENET, vol. 210, no. 2, December 1987 (1987-12), pages 331-337, XP001247107
- BARTKUS ET AL: "Isolation of a mutation resulting in constitutive synthesis of L-fucose catabolic enzymes" JOURNAL OF BACTERIOLOGY, vol. 165, no. 3, March 1986 (1986-03), pages 710-714, XP002394190

## Description

### Technical Field

The present invention relates to the microbiological industry, and specifically to a method for producing an L-amino acid using a bacterium of the *Enterobacteriaceae* family, which has been modified to enhance expression of genes comprising the *fucPIKUR* operon.

### Background Art

In *Escherichia coli,* the *fucPIKUR* operon is made up of five genes involved in utilization of L-fucose as a carbon and energy source. These genes include *fucP* (encoding L-fucose permease), *fucI* (encoding L-fucose isomerase), *fucK* (encoding L-fuculose kinase), *fucU* (encoding L-fucose-binding protein), and *fucR* (encoding the regulatory protein). The *fucPIKUR* operon is transcribed anticlockwise.

The *fucP* gene encodes the FucP protein, an L-fucose/proton symporter, which is also called as L-fucose permease, responsible for the uptake of L-fucose. FucP is the sole system of L-fucose transport in *Escherichia coli* (Bradley, S.A. et al., Biochem J., 1987, 248(2):495-500). The FucP protein crosses the cytoplasmic membrane of *E. coli* 12 times, with the N- and C-termini located in the cytoplasm (Gunn, F.J. et al., Mol. Microbiol., 1995, 15(4):771-783). The FucP protein is a member of the major facilitator superfamily (MFS), which is one of the two largest families of membrane transporters and is present ubiquitously in bacteria, archaea, and eukarya (Pao, S.S. et al., Microbiol. Mol. Biol. Rev., 1998, 62(1):1-34).

The *fucI* gene encodes L-fucose isomerase, an enzyme of the fucose catabolism pathway, which catalyzes conversion of L-fucose to L-fuculose (Green, M. and Cohen, S.S., J. Biol. Chem., 1956, 219(2):557-568; Elsinghorst, E.A. and Mortlock, R.P., J. Bacteriol., 1994, 176(23):7223-7232).

The *fucK* gene encodes L-fuculose kinase (L-fuculokinase), which is an enzyme of the fucose catabolism pathway catalyzing phosphorylation of L-fuculose (Elsinghorst, E.A. and Mortlock, R.P., J. Bacteriol., 1994,176(23):7223-7232).

The FucU protein encoded by the *fucU* gene is a cytoplasmic L-fucose binding protein lacking an enzyme activity toward L-fucose. It was suggested that FucU may play a role in fucose transport (Kim, M.S. et al., J. Biol. Chem., 2003, 278(30):28173-28180). Utilizing NMR techniques, FucU was shown to catalyze the anomeric conversion of fucose (Ryu, K.S. et al., J. Biol. Chem., 2004, 279(24):25544-25548).

Expression of the *fucPIKUR* operon is controlled by the FucR protein encoded by the *fucR* gene. FucR is a transcriptional activator that belongs to the DeoR family of transcriptional regulators (Chen, Y.M. et al., Mol. Gen. Genet., 1987, 210(2):331-337; Chen, Y.M. et al., J. Bacteriol., 1989, 171(11):6097-6105).

Currently, there have been no reports of enhancing expression of the *fucPIKUR* operon genes for the purpose of producing L-amino acids. WO 2004 90 125 discloses a method for producing L-amino acids using bacteria having enhanced expression of the PCKA gene.

### Disclosure of the Invention

Objects of the present invention include enhancing the productivity of L-amino acid-producing strains and providing a method for producing an L-amino acid using these strains.

The above objects were achieved by finding that enhancing expression of the *fucPIKUR* operon can increase production of L-amino acids, such as L-threonine, L-lysine, L-cysteine, L-methionine, L-leucine, L-isoleucine, L-valine, L-histidine, glycine, L-serine, L-alanine, L-asparagine, L-aspartic acid, L-glutamine, L-glutamic acid, L-proline, L-arginine, L-phenylalanine, L-tyrosine, and L-tryptophan.

The present invention provides a method for producing an L-amino acid comprising cultivating an L-amino acid-producing bacteria of the Entero-bacteriaceae family, wherein said bacterium has been modified to enhance expression of at least one gene of the fucPIKUR operon in a medium to produce and excrete said L-amino acid into the medium, and collecting said L-amino acid from the medium.

It is a further object of the present invention to provide the method as described above, wherein said gene expression is enhanced by modifying an expression control sequence for the *fucPIKUR* operon.

It is a further object of the present invention to provide the method as described above, wherein said gene expression is enhanced by increasing the copy number for at least one gene of the *fucPIKUR* operon.

It is a further object of the present invention to provide the method as described above, wherein the bacterium belongs to the genus *Escherichia.*

It is a further object of the present invention to provide the method as described above, wherein the bacterium belongs to the genus *Pantoea.*

It is a further object of the present invention to provide the method as described above, wherein said L-amino acid is selected from the group consisting of an aromatic L-amino acid and a non-aromatic L-amino acid.

It is a further object of the present invention to provide the method as described above, wherein said aromatic L-amino acid is selected from the group consisting of L-phenylalanine, L-tyrosine, and L-tryptophan.

It is a further object of the present invention to provide the method as described above, wherein said non-aromatic L-amino acid is selected from the group consisting of L-threonine, L-lysine, L-cysteine, L-methionine, L-leucine, L-isoleucine, L-valine, L-histidine, glycine, L-serine, L-alanine, L-asparagine, L-aspartic acid, L-glutamine, L-glutamic acid, L-proline, and L-arginine.

The present invention is described in detail below.

### Detailed Description of the Preferred Embodiments

### 1. Bacterium of the present invention

The bacterium of the present invention is an L-amino acid-producing bacterium of the *Enterobacteriaceae* family, wherein the bacterium has been modified to enhance expression of at least one gene involved in utilization of fucose, especially expression of the *fucPIKUR* operon.

In the present invention, "L-amino acid-producing bacterium" means a bacterium which has an ability to produce and excrete an L-amino acid into a medium, when the bacterium is cultured in the medium.

The term "L-amino acid-producing bacterium" as used herein also means a bacterium which is able to produce and cause accumulation of an L-amino acid in a culture medium in an amount larger than a wild-type or parental strain of the bacterium, for example, *E. coli,* such as *E. coli* K-12, and preferably means that the bacterium is able to cause accumulation in a medium of an amount not less than 0.5 g/L, more preferably not less than 1.0 g/L, of the target L-amino acid. The term "L-amino acid" includes L-alanine, L-arginine, L-asparagine, L-aspartic acid, L-cysteine, L-glutamic acid, L-glutamine, glycine, L-histidine, L-isoleucine, L-leucine, L-lysine, L-methionine, L-phenylalanine, L-proline, L-serine, L-threonine, L-tryptophan, L-tyrosine, and L-valine.

The term "aromatic L-amino acid" includes L-phenylalanine, L-tyrosine, and L-tryptophan. The term "non-aromatic L-amino acid" includes L-threonine, L-lysine, L-cysteine, L-methionine, L-leucine, L-isoleucine, L-valine, L-histidine, glycine, L-serine, L-alanine, L-asparagine, L-aspartic acid, L-glutamine, L-glutamic acid, L-proline, and L-arginine. L-threonine, L-lysine, L-cysteine, L-leucine, L-histidine, L-glutamic acid, L-phenylalanine, L-tryptophan, L-proline and L-arginine are particularly preferred.

The *Enterobacteriaceae* family includes bacteria belonging to the genera *Escherichia, Enterobacter, Erwinia, Klebsiella, Pantoea, Photorhabdus, Providencia, Salmonella, Serratia, Shigella, Morganella Yersinia,* etc. Specifically, those classified into the *Enterobacteriaceae* according to the taxonomy used by the NCBI (National Center for Biotechnology Information) database (http://www.ncbi.nlm.nih.gov/Taxonomy/Browser/wwwtax.cgi?id=91347) can be used. A bacterium belonging to the genus *Escherichia* or *Pantoea* is preferred.

The phrase "a bacterium belonging to the genus *Escherichia"* means that the bacterium is classified into the genus *Escherichia* according to the classification known to a person skilled in the art of microbiology. Examples of a bacterium belonging to the genus *Escherichia* as used in the present invention include, but are not limited to, *Escherichia coli (E. coli).*

The bacterium belonging to the genus *Escherichia* that can be used in the present invention is not particularly limited; however, e.g., bacteria described by Neidhardt, F.C. et al. (Escherichia coli and Salmonella typhimurium, American Society for Microbiology, Washington D.C., 1208, Table 1) are encompassed by the present invention.

The phrase "a bacterium belonging to the genus *Pantoea"* means that the bacterium is classified into the genus *Pantoea* according to the classification known to a person skilled in the art of microbiology. Some species of *Enterobacter agglomerans* have been recently re-classified into *Pantoea agglomerans, Pantoea ananatis, Pantoea stewartii* or the like, based on the nucleotide sequence analysis of 16S rRNA, etc (Int. J. Syst. Bacteriol., 43, 162-173 (1993)).

The phrase "bacterium has been modified to enhance expression of the *fucPIKUR* operon" means that the bacterium has been modified in such a way that the modified bacterium contains increased amounts of the proteins FucP, FucI, FucK, FucU, and FucR, as compared with an unmodified bacterium.

The phrase "the enhanced expression of the *fucPIKUR* operon" means that the expression level of genes comprising the *fucPIKUR* operon is higher than that of a non-modified strain, for example, a wild-type strain. Examples of such modification include increasing the copy number of the expressed gene(s) per cell, and/or increasing the expression level of the gene(s) by modification of an adjacent region of the gene, including sequences controlling gene expression, such as a promoter, enhancer, attenuator, ribosome-binding site, etc..

The *fucP* gene encodes the FucP protein, an L-fucose/proton symporter (synonym - B2801). The *fucP* gene *of E. coli* (nucleotide positions: 2,932,257 to 2,933,573; GenBank accession no. NC_000913.2; gi:49175990; SEQ ID NO: 1) is located between the *fucA* and *fucI* genes on the chromosome *of E. coli* K-12. The nucleotide sequence of the *fucP* gene and the amino acid sequence of the FucP protein encoded by the *fucP* gene are shown in SEQ ID NO: 1 and SEQ ID NO: 2, respectively.

The *fucI* gene encodes the FucI protein, which is an L-fucose isomerase (synonym - B2802). The *fucI* gene of *E. coli* (nucleotide positions: 2,933,606 to 2,935,381; GenBank accession no. NC_000913.2; gi:49175990; SEQ ID NO: 3) is located between the *fucP* and *fucK* genes on the chromosome of *E. coli* K-12. The nucleotide sequence of the *fucI* gene and the amino acid sequence of the FucI protein encoded by the *fucI* gene are shown in SEQ ID NO: 3 and SEQ ID NO: 4, respectively.

The *fucK* gene encodes the FucK protein, which is an L-fuculokinase (synonyms-B2803, ATP:L-fuculose 1-phosphotransferase). The *fucK* gene of *E. coli* (nucleotide positions: 2,935,460 to 2,936,908; GenBank accession no. NC_000913.2; gi:49175990; SEQ ID NO: 5) is located between the *fucI* and *fucU* genes on the chromosome of *E. coli* K-12. The nucleotide sequence of the *fucK* gene and the amino acid sequence of the FucK protein encoded by the *fucK* gene are shown in SEQ ID NO: 5 and SEQ ID NO: 6, respectively.

The *fucU* gene encodes the FucU protein, which is a cytoplasmic L-fucose-binding protein (synonym - B2804). The *fucU* gene *of E. coli* (nucleotide positions: 2,936,910 to 2,937,332; GenBank accession no. NC_000913.2; gi:49175990; SEQ ID NO: 7) is located between the *fucK* and *fucR* genes on the chromosome of *E. coli* K-12. The nucleotide sequence of the *fucU* gene and the amino acid sequence of the FucU protein encoded by the *fucU* gene are shown in SEQ ID NO: 7 and SEQ ID NO: 8, respectively.

The *fucR* gene encodes the FucR protein, which is a transcriptional activator (synonyms - B2805, positive regulator of the *fuc* operon). The *fucR* gene of *E. coli* (nucleotide positions: 2,937,390 to 2,938,121; GenBank accession no. NC_000913.2; gi:49175990; SEQ ID NO: 9) is located between the *fucU and ygdE* genes on the chromosome of *E. coli* K-12. The nucleotide sequence of the *fucR* gene and the amino acid sequence of the FucR protein encoded by the *fucR* gene are shown in SEQ ID NO: 9 and SEQ ID NO: 10, respectively.

The genes of the *fucPIKUR* operon can be obtained by PCR (polymerase chain reaction; refer to White, T.J. et al., Trends Genet., 5, 185 (1989)) utilizing primers prepared based on the known nucleotide sequence of the gene.

Since there may be some differences in DNA sequences between the genera or strains of the *Enterobacteriaceae* family, the above-described genes of the *fucPIKUR* operon to be overexpressed are not limited to the nucleotide sequences shown in SEQ ID NOS: 1, 3, 5, 7 and 9, but may also include nucleotide sequences homologous to SEQ ID NOS: 1, 3, 5, 7 and 9 encoding variants proteins of the FucP, FucI, FucK, FucU and FucR proteins, respecitively. The phrase "variant protein" as used in the present invention means a protein which has changes in the sequence, whether they are deletions, insertions, additions, or substitutions of one or several amino acids, but still maintains the activity of the product as the FucP, FucI, FucK, FucU or FucR protein. The number of changes in the variant protein depends on the position in the three dimensional structure of the protein or the type of amino acid residues. It may be 1 to 30, preferably 1 to 15, and more preferably 1 to 5 in SEQ ID NO: 2, 4, 6, 8 or 10. These changes in the variants can occur in regions of the protein which are not critical for the function of the protein. This is because some amino acids have high homology to one another so the three dimensional structure or activity is not affected by such a change. These changes in the variant protein can occur in regions of the protein which are not critical for the function of the protein. Therefore, the protein variants encoded by the above-described genes of the *fucPIKUR* operon may have a similarity (homology) of not less than 80%, preferably not less than 90%, and most preferably not less than 95%, with respect to the entire amino acid sequences shown in SEQ ID NOS. 2, 4, 6, 8 or 10, as long as the ability of the proteins to utilize L-fucose is maintained. Homology between two amino acid sequences can be determined using the well-known methods, for example, the computer program BLAST 2.0, which calculates three parameters: score, identity and similarity.

The substitution, deletion, insertion, or addition of one or several amino acid residues should be conservative mutation(s) so that the activity is maintained. The representative conservative mutation is a conservative substitution. Examples of conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val.

Moreover, each of the above-described genes of the *fucPIKUR* operon may be a variant which hybridizes under stringent conditions with the nucleotide sequence shown in SEQ ID NOS: 1, 3, 5, 7 or 9, or with a probe which can be prepared from the nucleotide sequence, provided that it encodes a functional protein. "Stringent conditions" include those under which a specific hybrid, for example, a hybrid having homology of not less than 60%, preferably not less than 70%, more preferably not less than 80%, still more preferably not less than 90%, and most preferably not less than 95%, is formed and a nonspecific hybrid, for example, a hybrid having homology lower than the above, is not formed. For example, stringent conditions are exemplified by washing at 60°C one time or more, preferably two or three times, at a salt concentration of 1 × SSC and 0.1% SDS, preferably 0.1 × SSC and 0.1% SDS at 60ºC. Duration of washing depends on the type of membrane used for blotting and, as a rule, may be what is recommended by the manufacturer. For example, the recommended duration of washing for the Hybond^{™} N⁺ nylon membrane (Amersham) under stringent conditions is 15 minutes. Preferably, washing may be performed 2 to 3 times. The length of the probe may be suitably selected, depending on the hybridization conditions, and usually varies from 100 bp to 1 kbp.

Methods of gene expression enhancement include increasing the gene copy number. Introduction of a recombinant plasmid comprising the gene and a vector that is able to function in a bacterium of the *Enterobacteriaceae* family increases the copy number of the gene. Low copy vectors and high copy vectors may be used. However, low copy vectors are preferably used. Examples of low-copy vectors include but are not limited to pSC101, pMW118, pMW119, and the like. The term "low copy vector" is used for vectors, the copy number of which is up to 5 copies per cell.

Enhancement of gene expression may also be achieved by introduction of multiple copies of the gene into the bacterial chromosome by, for example, a method of homologous recombination, Mu integration, or the like. For example, one act of Mu integration allows introduction of up to 3 copies of the gene into the bacterial chromosome.

Increasing the copy number of genes of the *fucPIKUR* operon can also be achieved by introducing multiple copies of the genes into the chromosomal DNA of the bacterium. In order to introduce multiple copies of the gene into the bacterial chromosome, homologous recombination is carried out using a sequence whose multiple copies exist as targets in the chromosomal DNA. Sequences having multiple copies in the chromosomal DNA include, but are not limited to, repetitive DNA, or inverted repeats existing at the end of a transposable element. Also, as disclosed in U.S. Patent No. 5,595,889, it is possible to incorporate genes of the *fucPIKUR* operon into a transposon, and allow it to be transferred to introduce multiple copies of the genes into the chromosomal DNA.

Enhancing gene expression may also be achieved by placing genes of the *fucPIKUR* operon under the control of a potent promoter which is preferably more potent than the inherent promoter of the operon. For example, the P_{tac} promoter, the *lac* promoter, the *trp* promoter, the *trc* promoter, the P_{R}, or the P_{L} promoters of lambda phage are all known to be potent promoters. The use of a potent promoter can be combined with multiplication of gene copies.

Alternatively, the effect of a promoter can be enhanced by, for example, introducing a mutation into the promoter to increase the transcription level of a gene located downstream of the promoter. Furthermore, it is known that substitution of several nucleotides in the spacer between ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629,1984). Previously, it was shown that the *rhtA23* mutation, which increases the resistance to threonine, homoserine and some other substances transported out of cells, is an A-for-G substitution at the -1 position relative to the ATG start codon (ABSTRACTS of 17^{th} International Congress of Biochemistry and Molecular Biology in conjugation with 1997 Annual Meeting of the American Society for Biochemistry and Molecular Biology, San Francisco, California August 24-29, 1997, abstract No. 457). Therefore, it may be suggested that the *rhtA23* mutation enhances translation of the *rhtA* gene transcript and, as a consequence, increases the resistance to the above-mentioned substances.

Moreover, it is also possible to introduce a nucleotide substitution into the expression control sequence such as a promoter region of the *fucPIKUR* operon on the bacterial chromosome, which results in stronger promoter function. The alteration of the expression control sequence can be performed, for example, in the same manner as the gene substitution using a temperature-sensitive plasmid, as disclosed in WO 00/18935 and JP 1-215280 A.

The level of gene expression can be estimated by measuring the amount of mRNA transcribed from the gene using various well-known methods, including Northern blotting, quantitative RT-PCR, and the like. The amount or molecular weight of the protein encoded by the gene can be measured by well-known methods, including SDS-PAGE followed by immunoblotting assay (Western blotting analysis) and the like.

Methods for preparation of plasmid DNA, digestion and ligation of DNA, transformation, selection of an oligonucleotide as a primer, and the like may be ordinary methods well-known to one skilled in the art. These methods are described, for instance, in Sambrook, J., Fritsch, E.F., and Maniatis, T., "Molecular Cloning: A Laboratory Manual, Second Edition", Cold Spring Harbor Laboratory Press (1989).

### L-amino acid-producing bacteria

As a bacterium of the present invention which is modified to enhance expression of the *fucPIKUR* operon, bacteria which are able to produce either an aromatic or a non-aromatic L-amino acid may be used.

The bacterium of the present invention can be obtained by enhancing expression of the *fucPIKUR* operon in a bacterium, which inherently has the ability to produce an L-amino acid. Alternatively, the bacterium of present invention can be obtained by imparting the ability to produce an L-amino acid to a bacterium already having the expression of the *fucPIKUR* operon enhanced.

### L-threonine-producing bacteria

Examples of parent strains for deriving the L-threonine-producing bacteria of the present invention include, but are not limited to, strains belonging to the genus *Escherichia,* such as *E*. *coli* TDH-6/pVIC40 (VKPM B-3996) (U.S. Patent No. 5, 175,107, U.S. Patent No. 5,705,371), *E. coli* 472T23/pYN7 (ATCC 98081) (U.S. Patent No.5,631,157), *E*. *coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E*. *coli* MG442 (Gusyatiner et al., Genetika (in Russian), 14, 947-956 (1978)), *E. coli* VL643 and VL2055 (EP 1149911 A), and the like.

The strain TDH-6 is deficient in the *thrC* gene, as well as being sucrose-assimilative, and the *ilvA* gene has a leaky mutation. This strain also has a mutation in the *rhtA* gene, which imparts resistance to high concentrations of threonine or homoserine. The strain B-3996 contains the plasmid pVIC40 which was obtained by inserting a *thrA *BC* operon which includes a mutant *thrA* gene into a RSF1010-derived vector. This mutant *thrA* gene encodes aspartokinase homoserine dehydrogenase I which has substantially desensitized feedback inhibition by threonine. The strain B-3996 was deposited on November 19,1987 in the All-Union Scientific Center of Antibiotics (Nagatinskaya Street 3-A, 117105 Moscow, Russian Federation) under the accession number RIA 1867. The strain was also deposited in the Russian National Collection of Industrial Microorganisms (VKPM) (Russia, 117545 Moscow, 1 Dorozhny proezd. 1) on April 7, 1987 under the accession number VKPM B-3996.

*E. coli* VKPM B-5318 (EP 0593792B) may also be used as a parent strain for deriving L-threonine-producing bacteria of the present invention. The strain B-5318 is prototrophic with regard to isoleucine, and a temperature-sensitive lambda-phage C1 repressor and PR promoter replaces the regulatory region of the threonine operon in plasmid pVIC40. The strain VKPM B-5318 was deposited in the Russian National Collection of Industrial Microorganisms (VKPM) on May 3, 1990 under accession number of VKPM B-5318.

Preferably, the bacterium of the present invention is additionally modified to enhance expression of one or more of the following genes:
- the mutant *thrA* gene which codes for aspartokinase homoserine dehydrogenase I resistant to feed back inhibition by threonine;
- the *thrB* gene which codes for homoserine kinase;
- the *thrC* gene which codes for threonine synthase;
- the *rhtA* gene which codes for a putative transmembrane protein;
- the *asd* gene which codes for aspartate-β-semialdehyde dehydrogenase; and
- the *aspC* gene which codes for aspartate aminotransferase (aspartate transaminase);

The *thrA* gene which encodes aspartokinase homoserine dehydrogenase I of *Escherichia coli* has been elucidated (nucleotide positions 337 to 2799, GenBank accession NC_000913.2, gi: 49175990). The *thrA* gene is located between the *thrL* and *thrB* genes on the chromosome of *E. coli* K-12. The *thrB* gene which encodes homoserine kinase of *Escherichia coli* has been elucidated (nucleotide positions 2801 to 3733, GenBank accession NC_000913.2, gi: 49175990). The *thrB* gene is located between the *thrA* and *thrC* genes on the chromosome of *E*. *coli* K-12. The *thrC* gene which encodes threonine synthase of *Escherichia coli* has been elucidated (nucleotide positions 3734 to 5020, GenBank accession NC_000913.2, gi: 49175990). The *thrC* gene is located between the *thrB* gene and the *yaaX* open reading frame on the chromosome of *E. coli* K-12. All three genes functions as a single threonine operon. To enhance expression of the threonine operon, the attenuator region which affects the transcription is desirably removed from the operon (WO2005/049808, WO2003/097839).

A mutant *thrA* gene which codes for aspartokinase homoserine dehydrogenase I resistant to feed back inhibition by threonine, as well as, the *thrB* and *thrC* genes can be obtained as one operon from well-known plasmid pVIC40 which is presented in the threonine producing *E*. *coli* strain (VKPM B-3996. Plasmid pVIC40 is described in detail in U.S. Patent No. 5,705,371.

The *rhtA* gene exists at 18 min on the E. coli chromosome close to the *glnHPQ* operon, which encodes components of the glutamine transport system. The *rhtA* gene is identical to ORF1 (*ybiF* gene, nucleotide positions 764 to 1651, GenBank accession number AAA218541, gi:440181) and located between the *pexB* and *ompX* genes. The unit expressing a protein encoded by the ORF1 has been designated the *rht4* gene (rht: resistance to homoserine and threonine). Also, it was revealed that the rhtA23 mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A).

The *asd* gene of *E. coli* has already been elucidated (nucleotide positions 3572511 to 3571408, GenBank accession NC_000913.1, gi:16131307), and can be obtained by PCR (polymerase chain reaction; refer to White, T.J. et al., Trends Genet., 5, 185 (1989)) utilizing primers prepared based on the nucleotide sequence of the gene. The *asd* genes of other microorganisms can be obtained in a similar manner.

Also, the *aspC* gene of *E. coli* has already been elucidated (nucleotide positions 983742 to 984932, GenBank accession NC_000913.1, gi:16128895), and can be obtained by PCR. The *aspC* genes of other microorganisms can be obtained in a similar manner.

### L-lysine-producing bacteria

Examples of L-lysine-producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine producing bacterium of *Escherichia coli.* This bacterial strain was bred by conferring AEC resistance to the strain W3110, which was derived from *Escherichia coli* K-12. The resulting strain was designated *Escherichia coli* AJ13069 strain and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of parent strains for deriving L-lysine-producing bacteria of the present invention also include strains in which expression of one or more genes encoding an L-lysine biosynthetic enzyme are enhanced. Examples of such genes include, but are not limited to, genes encoding dihydrodipicolinate synthase (*dapA*)*,* aspartokinase (*lysC*)*,* dihydrodipicolinate reductase (*dapB*), diaminopimelate decarboxylase (*lysA*), diaminopimelate dehydrogenase (ddh) (U.S. Patent No. 6,040,160), phosphoenolpyrvate carboxylase (*ppc*)*,* aspartate semialdehyde dehydrogenease (*asd*)*,* and aspartase (*aspA*) (EP 1253195 A). In addition, the parent strains may have an increased level of expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (WO2005/073390), or combinations thereof.

Examples of parent strains for deriving L-lysine-producing bacteria of the present invention also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (WO2005/010175).

### L-cysteine-producing bacteria

Examples of parent strains for deriving L-cysteine-producing bacteria of the present invention include, but are not limited to, strains belonging to the genus *Escherichia,* such as *E. coli* JM15 which is transformed with different *cysE* alleles coding for feedback-resistant serine acetyltransferases (U.S. Patent No. 6,218,168, Russian patent application 2003121601); *E. coli* W3110 having over-expressed genes which encode proteins suitable for secreting substances toxic for cells (U.S. Patent No. 5,972,663); *E. coli* strains having lowered cysteine desulfohydrase activity (JP11155571A2); *E. coli* W3110 with increased activity of a positive transcriptional regulator for cysteine regulon encoded by the *cysB* gene (WO0127307A1), and the like.

### L-leucine-producing bacteria

Examples of parent strains for deriving L-leucine-producing bacteria of the present invention include, but are not limited to, strains belonging to the genus *Escherichia,* such as *E. coli* strains resistant to leucine (for example, the strain 57 (VKPM B-7386, U.S. Patent No. 6,124,121)) or leucine analogs including β-2-thienylalanine, 3-hydroxyleucine, 4-azaleucine, 5,5,5-trifluoroleucine (JP 62-34397 B and JP 8-70879 A); *E. coli* strains obtained by the gene engineering method described in WO96/06926; *E. coli* H-9068 (JP 8-70879 A), and the like.

The bacterium, of the present invention may be improved by enhancing the expression of one or more genes involved in L-leucine biosynthesis. Examples include genes of the *leuABCD* operon, which are preferably represented by a mutant *leuA* gene coding for isopropylmalate synthase freed from feedback inhibition by L-leucine (US Patent 6,403,342). In addition, the bacterium of the present invention may be improved by enhancing the expression of one or more genes coding for proteins which excrete L-amino acid from the bacterial cell. Examples of such genes include the b2682 and b2683 genes (*ygaZH* genes) (EP 1239041 A2).

### L-histidine-producing bacteria

Examples of parent strains for deriving L-histidine-producing bacteria of the present invention include, but are not limited to, strains belonging to the genus *Escherichia,* such as *E. coli* strain 24 (VKPM B-5945,' RU2003677); *E. coli* strain 80 (VKPM B-7270, RU2119536); *E. coli* NRRL B-12116 - B12121 (U.S. Patent No. 4,388,405); *E. coli* H-9342 (FERM BP-6675) and H-9343 (FERM BP-6676) (U.S. Patent No. 6,344,347); *E. coli* H-9341 (FERM BP-6674) (EP1085087); *E. coli* AI80/pFM201 (U,S. Patent No. 6,258,554) and the like.

Examples of parent strains for deriving L-histidine-producing bacteria of the present invention also include strains in which expression of one or more genes encoding an L-histidine biosynthetic enzyme are enhanced. Examples of such genes include genes encoding ATP phosphoribosyltransferase (*hisG*), phosphoribosyl AMP cyclohydrolase *(hisI),* phosphoribosyl-ATP pyrophosphohydrolase (*hisIE*), phosphoribosylformimino-5-aminoimidazole carboxamide ribotide isomerase *(hisA),* amidotransferase *(hisH),* histidinol phosphate aminotransferase *(hisC),* histidinol phosphatase *(hisB),* histidinol dehydrogenase *(hisD),* and so forth.

It is known that the L-histidine biosynthetic enzymes encoded by *hisG* and *hisBHAFI* are inhibited by L-histidine, and therefore an L-histidine-producing ability can also be efficiently enhanced by introducing a mutation conferring resistance to the feedback inhibition into ATP phosphoribosyltransferase (Russian Patent Nos. 2003677 and 2119536).

Specific examples of strains having an L-histidine-producing ability include *E. coli* FERM-P 5038 and 5048 which have been introduced with a vector carrying a DNA encoding an L-histidine-biosynthetic enzyme (JP 56-005099 A), *E. coli* strains introduced with *rht,* a gene for an amino acid-export (EP1016710A), *E. coli* 80 strain imparted with sulfaguanidine, DL-1,2,4-triazole-3-alanine, and streptomycin-resistance (VKPM B-7270, Russian Patent No. 2119536), and so forth.

### L-glutamic acid-producing bacteria

Examples of parent strains for deriving L-glutamic acid-producing bacteria of the present invention include, but are not limited to, strains belonging to the genus *Escherichia,* such as *E. coli* VL334thrC⁺ (EP 1172433). *E. coli* VL334 (VKPM B-1641) is an L-isoleucine and L-threonine auxotrophic strain having mutations in *thrC* and *ilvA* genes (U.S. Patent No. 4,278,765). A wild-type allele of the *thrC* gene was transferred by the method of general transduction using a bacteriophage P1 grown on the wild-type *E. coli* strain K12 (VKPM B-7) cells. As a result, an L-isoleucine auxotrophic strain VL334thrC⁺ (VKPM B-8961), which is able to produce L-glutamic acid, was obtained.

Examples of parent strains for deriving the L-glutamic acid-producing bacteria of the present invention include, but are not limited to, strains in which expression of one or more genes encoding an L-glutamic acid biosynthetic enzyme are enhanced. Examples of such genes include genes encoding glutamate dehydrogenase (*gdh*), glutamine synthetase (*glnA*), glutamate synthetase (*gltAB*)*,* isocitrate dehydrogenase (*icdA*)*,* aconitate hydratase *(acnA, acnB),* citrate synthase (*glt*A), phosphoenolpyruvate carboxylase (*ppc*)*,* pyruvate dehydrogenase (*aceEF, lpdA*)*,* pyruvate kinase (*pykA, pykF*)*,* phosphoenolpyruvate synthase (*ppsA*), enolase (*eno*), phosphoglyceromutase (*pgmA, pgmI*)*,* phosphoglycerate kinase (*pgk*)*,* glyceraldehyde-3-phophate dehydrogenase (*gapA*), triose phosphate isomerase (*tpiA*), fructose bisphosphate aldolase (*fbp*), phosphofructokinase (*pfkA, pfkB),* and glucose phosphate isomerase (*pgi*).

Examples of strains modified so that expression of the citrate synthetase gene, the phosphoenolpyruvate carboxylase gene, and/or the glutamate dehydrogenase gene is/are enhanced include those disclosed in EP1078989A, EP955368A, and EP952221A.

Examples of parent strains for deriving the L-glutamic acid-producing bacteria of the present invention also include strains having decreased or eliminated activity of an enzyme that catalyzes synthesis of a compound other than L-glutamic acid by branching off from an L-glutamic acid biosynthesis pathway. Examples of such genes include genes encoding isocitrate lyase (*aceA*), α-ketoglutarate dehydrogenase (*sucA*)*,* phosphotransacetylase (*pta*)*,* acetate kinase (*ack*)*,* acetohydroxy acid synthase (*ilvG*)*,* acetolactate synthase (*ilvI*)*,* formate acetyltransferase (*pfl*), lactate dehydrogenase (*ldh*), and glutamate decarboxylase (*gadAB*). Bacteria belonging to the genus *Escherichia* deficient in the α-ketoglutarate dehydrogenase activity or having a reduced α-ketoglutarate dehydrogenase activity and methods for obtaining them are described in U.S. Patent Nos. 5,378,616 and 5,573,945. Specifically, these strains include the following:
*E. coli* W3110sucA::Kmr
*E. coli* AJ12624 (FERM BP-3853)
*E. coli* AJ12628 (FERM BP-3854)
*E. coli* AJ12949 (FERM BP-4881)

*E. coli* W3110sucA::Kmr is a strain obtained by disrupting the α-ketoglutarate dehydrogenase gene (hereinafter referred to as "*sucA* gene") *of E. coli* W3110. This strain is completely deficient in the α-ketoglutarate dehydrogenase.

Other examples of L-glutamic acid-producing bacterium include those which belong to the genus *Escherichia* and have resistance to an aspartic acid antimetabolite. These strains can also be deficient in the α-ketoglutarate dehydrogenase activity and include, for example, *E. coli* AJ 13199 (FERM BP-5807) (U.S. Patent No. 5.908,768), FFRM P-12379, which additionally has a low L-glutamic acid decomposing ability (U.S. Patent No. 5,393,671); AJ13138 (FERM BP-5565) (U.S. Patent No. 6,110,714), and the like.

Examples of L-glutamic acid-producing bacteria, include mutant strains belonging to the genus *Pantoea* which are deficient in the α-ketoglutarate dehydrogenase activity or have a decreased α-ketoglutarate dehydrogenase activity, and can be obtained as described above. Such strains include *Pantoea ananatis* AJ13356. (U.S. Patent No. 6,331,419). *Pantoea ananatis* AJ13356 was deposited at the National Institute of Bioscience and Human-Technology, Agency of Industrial Science and Technology, Ministry of International Trade and Industry (currently, National Institute of Advanced Industrial Science and Technology, International Patent Organism Depositary, Central 6, 1-1, Higashi 1-Chome, Tsukuba-shi, Ibaraki-ken, 305-8566, Japan) on February 19, 1998 under an accession number of FERM P-16645. It was then converted to an international deposit under the provisions of Budapest Treaty on January 11, 1999 and received an accession number of FERM BP-6615.*Pantoea ananatis* AJ13356 is deficient in the α-ketoglutarate dehydrogenase activity as a result of disruption of the αKGDH-E1 subunit gene (sucA). The above strain was identified as *Enterobacter agglomerans* when it was isolated and deposited as the *Enterobacter agglomerans* AJ13356. However, it was recently re-classified as *Pantoea ananatis* on the basis of nucleotide sequencing of 16S-rRNA and so forth. Although AJ13356 was deposited at the aforementioned depository as *Enterobacter agglomerans,* for the purposes of this specification, they are described as *Pantoea ananatis.*

### L-phenylalanine-producing bacteria

Examples of parent strains for deriving L-phenylalanine-producing bacteria of the present invention include, but are not limited to, strains belonging to the genus *Escherichia,* such as *E. coli* AJ12739 (tyrA::Tn10, tyrR) (VKPM B-8197); *E. coli* HW1089 (ATCC 55371) harboring the mutant *pheA34* gene (U.S. Patent No. 5,354,672); *E. coli* MWEC101-b (KR8903681); *E. coli* NRRL B-12141, NRRL B-12145, NRRL B-12146 and NRRL B-12147 (U.S. Patent No. 4,407,952). Also, as a parent strain, *E. coli* K-12 [W3110 (tyrA)/pPHAB (FERM BP-3566), *E. coli* K-12 [W3110 (tyrA)/pPHAD] (FERM BP-12659), *E. coli* K-12 [W3110 (tyrA)/pPHATerm] (FERM BP-12662) and *E. coli* K-12 [W3110 (tyrA)/pBR-aroG4, pACMAB] named as AJ 12604 (FERM BP-3579) may be used (EP 488424 B1). Furthermore, L-phenylalanine producing bacteria belonging to the genus *Escherichia* with an enhanced activity of the protein encoded by the *yedA* gene or the *yddG* gene may also be used (U.S. patent applications 2003/0148473 A1 and 2003/0157667 A1).

### L-tryptophan-producing bacteria

Examples of parent strains for deriving the L-tryptophan-producing bacteria of the present invention include, but are not limited to, strains belonging to the genus *Escherichia,* such as *E. coli* JP4735/pMU3028 (DSM10122) and JP6015/pMU91 (DSM10123) which is deficient in the tryptophanyl-tRNA synthetase encoded by mutant *trpS* gene (U.S. Patent No. 5,756,345); *E. coli* SV164 (pGH5) having a *serA* allele encoding phosphoglycerate dehydrogenase free from feedback inhibition by serine and a *trpE* allele encoding anthranilate synthase free from feedback inhibition by tryptophan (U.S. Patent No. 6,180,373); *E. coli* AGX17 (pGX44) (NRRL B-12263) and AGX6(pGX50)aroP (NRRL B-12264) which is deficient in the enzyme tryptophanase (U.S. Patent No. 4,371,614); *E. coli* AGX17/pGX50,pACKG4-pps in which a phosphoenolpyruvate-producing ability is enhanced (WO9708333, U.S. Patent No. 6,319,696), and the like may be used. L-tryptophan-producing bacteria belonging to the genus *Escherichia* with an enhanced activity of the protein encoded by the *yedA* gene or the *yddG* gene may also be used (U.S. patent applications 2003/0148473 A1 and 2003/0157667 A1).

Examples of parent strains for deriving the L-tryptophan-producing bacteria of the present invention also include strains in which one or more activities of the enzymes selected from anthranilate synthase, phosphoglycerate dehydrogenase, and tryptophan synthase are enhanced. The anthranilate synthase and phosphoglycerate dehydrogenase are both subject to feedback inhibition by L-tryptophan and L-serine, so that a mutation desensitizing the feedback inhibition may be introduced into these enzymes. Specific examples of strains having such a mutation include a *E*. *coli* SV164 which harbors desensitized anthranilate synthase and a transformant strain obtained by introducing into the *E*. *coli* SV164 the plasmid pGH5 (WO 94/08031), which contains a mutant *serA* gene encoding feedback-desensitized phosphoglycerate dehydrogenase.

Examples of parent strains for deriving the L-tryptophan-producing bacteria of the present invention also include strains into which the tryptophan operon which contains a gene encoding desensitized anthranilate synthase has been introduced (JP 57-71397 A, JP 62-244382 A, U.S. Patent No. 4,371,614). Moreover, L-tryptophan-producing ability may be imparted by enhancing expression of a gene which encodes tryptophan synthase, among tryptophan operons *(trpBA).* The tryptophan synthase consists of α and β subunits which are encoded by the *trpA* and *trpB* genes, respectively. In addition, L-tryptophan-producing ability may be improved by enhancing expression of the isocitrate lyase-malate synthase operon (WO2005/103275).

### L-proline-producing bacteria

Examples of parent strains for deriving L-proline-producing bacteria of the present invention include, but are not limited to, strains belonging to the genus *Escherichia,* such as *E. coli* 702ilvA (VKPM B-8012) which is deficient in the *ilvA* gene and is able to produce L-proline (EP 1172433). The bacterium of the present invention may be improved by enhancing the expression of one or more genes involved in L-proline biosynthesis. Examples of such genes for L-proline producing bacteria which are preferred include the *proB* gene coding for glutamate kinase of which feedback inhibition by L-proline is desensitized (DE Patent 3127361). In addition, the bacterium of the present invention may be improved by enhancing the expression of one or more genes coding for proteins excreting L-amino acid from bacterial cell. Such genes are exemplified by b2682 and b2683 genes (*ygaZH* genes) (EP1239041 A2).

Examples of bacteria belonging to the genus *Escherichia,* which have an activity to produce L-proline include the following *E*. *coli* strains: NRRL B-12403 and NRRL B-12404 (GB Patent 2075056), VKPM B-8012 (Russian patent application 2000124295), plasmid mutants described in DE Patent 3127361, plasmid mutants described by Bloom F.R. et al (The 15^{th} Miami winter symposium, 1983, p.34), and the like.

### L-arginine-producing bacteria

Examples of parent strains for deriving L-arginine-producing bacteria of the present invention include, but are not limited to, strains belonging to the genus *Escherichia,* such as *E. coli* strain 237 (VKPM B-7925) (U.S. Patent Application 2002/058315 A1) and its derivative strains harboring mutant N-acetylglutamate synthase (Russian Patent Application No. 2001112869), *E. coli* strain 382 (VKPM B-7926) (EP1170358A1), an arginine-producing strain into which *argA* gene encoding N-acetylglutamate synthetase is introduced therein (EP1170361A1), and the like.

Examples of parent strains for deriving L-arginine producing bacteria of the present invention also include strains in which expression of one or more genes encoding an L-arginine biosynthetic enzyme are enhanced. Examples of such genes include genes encoding N-acetylglutamyl phosphate reductase (*argC*)*,* ornithine acetyl transferase (*argJ*)*,* N-acetylglutamate kinase (*argB*)*,* acetylornithine transaminase (*argD*)*,* ornithine carbamoyl transferase (*argF*)*,* argininosuccinic acid synthetase *(argG),* argininosuccinic acid lyase (*argH*)*,* and carbamoyl phosphate synthetase (*carAB*)*.*

### L-valine-producing bacteria

Example of parent strains for deriving L-valine-producing bacteria of the present invention include, but are not limited to, strains which have been modified to overexpress the *ilvGMEDA* operon (U.S. Patent No. 5,998,178). It is desirable to remove the region of the *ilvGMEDA* operon which is required for attenuation so that expression of the operon is not attenuated by L-valine that is produced. Furthermore, the *ilvA* gene in the operon is desirably disrupted so that threonine deaminase activity is decreased.

Examples of parent strains for deriving L-valine-producing bacteria of the present invention include also include mutants having a mutation of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 has been deposited in the Russian National Collection of Industrial Microorganisms (VKPM) (Russia, 113545 Moscow, 1 Dorozhny Proezd, 1) on June 24, 1988 under accession number VKPM B-4411.

Furthermore, mutants requiring lipoic acid for growth and/or lacking H⁺-ATPase can also be used as parent strains (WO96/06926).

### L-isoleucine-producing bacteria

Examples of parent strains for deriving L-isoleucine producing bacteria of the present invention include, but are not limited to, mutants having resistance to 6-dimethylaminopurine (JP 5-304969 A), mutants having resistance to an isoleucine analogue such as thiaisoleucine and isoleucine hydroxamate, and mutants additionally having resistance to DL-ethionine and/or arginine hydroxamate (JP 5-130882 A). In addition, recombinant strains transformed with genes encoding proteins involved in L-isoleucine biosynthesis, such as threonine deaminase and acetohydroxate synthase, can also be used as parent strains (JP 2-458 A, FR 0356739, and U.S. Patent No. 5,998,178).

### 2. Method of the present invention

The method of the present invention is a method for producing an L-amino acid comprising cultivating the bacterium of the present invention in a culture medium to produce and excrete the L-amino acid into the medium, and collecting the L-amino acid from the medium.

In the present invention, the cultivation, collection, and purification of an L-amino acid from the medium and the like may be performed in a manner similar to conventional fermentation methods wherein an amino acid is produced using a bacterium.

A medium used for culture may be either a synthetic or natural medium, so long as the medium includes a carbon source and a nitrogen source and minerals and, if necessary, appropriate amounts of nutrients which the bacterium requires for growth. The carbon source may include various carbohydrates such as glucose and sucrose, and various organic acids. Depending on the mode of assimilation of the used microorganism, alcohol, including ethanol and glycerol, may be used. As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used. As vitamins, thiamine, yeast extract, and the like, can be used.

The cultivation is preferably performed under aerobic conditions, such as a shaking culture, and a stirring culture with aeration, at a temperature of 20 to 40 °C, preferably 30 to 38 °C. The pH of the culture is usually between 5 and 9, preferably between 6.5 and 7.2. The pH of the culture can be adjusted with ammonia, calcium carbonate, various acids, various bases, and buffers. Usually, a 1 to 5-day cultivation leads to accumulation of the target L-amino acid in the liquid medium.

After cultivation, solids such as cells can be removed from the liquid medium by centrifugation or membrane filtration, and then the L-amino acid can be collected and purified by ion-exchange, concentration, and/or crystallization methods.

### Brief Description of Drawings

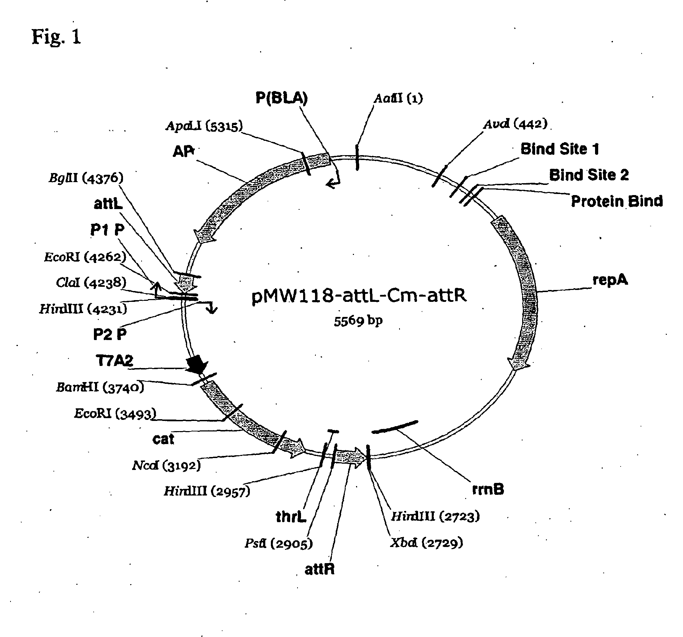

Figure 1 shows the construction of the pMW118-attL-Cm-attR plasmid used as a template for PCR.

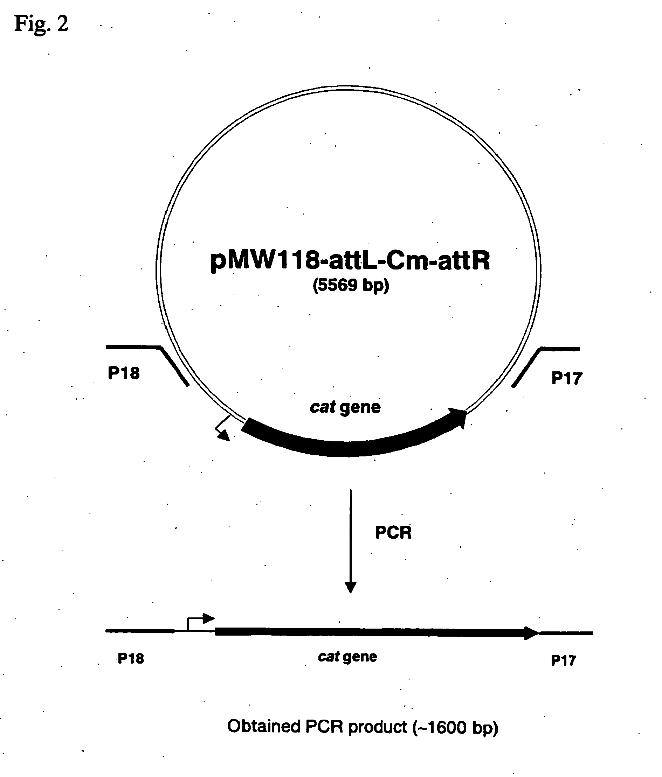

Figure 2 shows the relative positions of primers P17 and P18 on plasmid pMW118-attL-Cm-attR used for PCR amplification of the *cat* gene.
Figure 3 shows the construction of the chromosomal DNA fragment containing the hybrid P_{L-tac} promoter.
Figure 4 shows the effect of enhanced expression of the *fucPIKUR* operon on growth of the *E. coli* strain having the PTS transport system disrupted.

### Examples

The present invention will be more concretely explained below with reference to the following non-limiting Examples.

### Example 1. Preparation of the PCR template and helper plasmids

The PCR template plasmid pMW118-attL-Cm-attR and the helper plasmid pMW-intxis-ts were prepared as follows:

### (1) pMW118-attL-Cm-attR

The pMW118-attL-Cm-attR plasmid was constructed on the basis of pMW118-attL-Tc-attR that was obtained by ligation of the following four DNA fragments:
1) the *Bgl*II*-EcoR*I fragment (114 bp) carrying *attL* (SEQ ID NO: 11) which was obtained by PCR amplification of the corresponding region of the *E. coli* W3350 (contained λ prophage) chromosome using oligonucleotides P1 and P2 (SEQ ID NOS: 12 and 13) as primers (these primers contained the subsidiary recognition sites for *Bgl*II and *EcoR*I endonucleases);
2) the *Pst*I*-Hind*III fragment (182 bp) carrying *attR* (SEQ ID NO: 14) which was obtained by PCR amplification of the corresponding region of the *E. coli* W3350 (contained λ prophage) chromosome using the oligonucleotides P3 and P4 (SEQ ID NOS: 15 and 16) as primers (these primers contained the subsidiary recognition sites for *Pst*I and *Hind*III endonucleases);
3) the large *Bgl*II*-Hind*III fragment (3916 bp) of pMW118-ter_*rrn*B. The plasmid pMW118-ter_*rrnB* was obtained by ligation of the following three DNA fragments:
   - the large DNA fragment (2359 bp) carrying the *Aat*II*-EcoR*I fragment of pMW118 that was obtained by the following way: pMW118 was digested with *Eco*RI restriction endonuclease, treated with Klenow fragment of DNA polymerase I, and then digested with *Aat*II restriction endonuclease;
   - the small *Aat*II*-Bgl*II fragment (1194 bp) of pUC19 carrying the *bla* gene for ampicillin resistance (Ap^{R}) was obtained by PCR amplification of the corresponding region of the pUC19 plasmid using oligonucleotides P5 and P6 (SEQ ID NOS: 17 and 18) as primers (these primers contained the subsidiary recognition sites for *Aat*II and *Bgl*II endonucleases);
   - the small *Bgl*II*-Pst*I fragment (363 bp) of the transcription terminator ter_*rrnB* was obtained by PCR amplification of the corresponding region of the *E. coli* MG1655 chromosome using the oligonucleotides P7 and P8 (SEQ ID NOS: 19 and 20) as primers (these primers contained the subsidiary recognition sites for *Bgl*II and *Pst*I endonucleases);
4) the small *EcoR*I*-Pst*I fragment (1388 bp) (SEQ ID NO: 21) of pML-Tc-ter_*thrL* bearing the tetracycline resistance gene and the ter_*thrL* transcription terminator; the pML-Tc-ter*_thrL* plasmid was obtained in two steps:
   - the pML-ter_*thrL* plasmid was obtained by digesting the pML-MCS plasmid (Mashko, S.V. et al., Biotekhnologiya (in Russian), 2001, no. 5, 3-20) with the *Xba*I and *BamH*I restriction endonucleases followed by ligation of the large fragment (3342 bp) with the *Xba*I*-BamH*I fragment (68 bp) carrying terminator ter_*thrL* obtained by PCR amplification of the corresponding region of the *E*. *coli* MG1655 chromosome using oligonucleotides P9 and P10 (SEQ ID NOS: 22 and 23) as primers (these primers contained the subsidiary recognition sites for the *Xba*I and *BamH*I endonucleases);
   - the pML-Tc-ter_*thr*L plasmid was obtained by digesting the pML-ter_*thrL* plasmid with the *Kpn*I and *Xba*I restriction endonucleases followed by treatment with Klenow fragment of DNA polymerase I and ligation with the small *EcoR*I*-Van9l*I fragment (1317 bp) of pBR322 bearing the tetracycline resistance gene (pBR322 was digested with *EcoR*I and *Van91*I restriction endonucleases and then treated with Klenow fragment of DNA (polymerase I);

The above strain *E. coli* W3350 is a derivative of wild type strain *E. coli* K-12. The strain *E. coli* MG1655 (ATCC 700926) is a wild type strain and can be obtained from American Type Culture Collection (P.O. Box 1549 Manassas, VA 20108, United States of America). The plasmid pMW118 and pUC19 are commercially available. The *Bgl*II-*EcoR*I fragment carrying *attL* and the *Bgl*II*-Pst*I fragment of the transcription terminator ter_*rrnB* can be obtained from the other strain of *E*. *coli* in the same manner as describe above.

The pMW118-attL-Cm-attR plasmid was constructed by ligation of the large *BamH*I*-Xba*I fragment (4413 bp) of pMW118-attL-Tc-attR and the artificial DNA *Bgl*II-*Xba*I fragment (1162 bp) containing the P_{A2} promoter (the early promoter of the phage T7), the *cat* gene for chloramphenicol resistance (Cm^{R}), the ter_*thrL* transcription terminator, and attR. The artificial DNA fragment (SEQ ID NO: 24) was obtained by the following way:
1. The pML-MCS plasmid was digested with the *Kpn*I and *Xba*I restriction endonucleases and ligated with the small *Kpn*I*-Xba*I fragment (120 bp), which included the P_{A2} promoter (the early promoter of phage T7) obtained by PCR amplification of the corresponding DNA region of phage T7 using oligonucleotides P11 and P12 (SEQ ID NOS: 25 and 26, respectively) as primers (these primers contained the subsidiary recognition sites for *Kpn*I and *Xba*I endonucleases). As a result, the pML-P_{A2}-MCS plasmid was obtained. Complete nucleotide sequence of phage T7 has been reported (J. Mol. Biol., 166: 477-535 (1983).
2. The *Xba*I site was deleted from pML-P_{A2}-MCS. As a result, the pML-P_{A2}-MCS(*XbaI*⁻) plasmid was obtained.
3. The small *Bgl*II*-Hind*III fragment (928 bp) of pML-P_{A2}-MCS(XbaI⁻) containing the P_{A2} promoter (the early promoter of the phage T7) and the *cat* gene for chloramphenicol resistance (Cm^{R}) was ligated with the small *Hind*III-*Hind*III fragment (234 bp) of pMW118-attL-Tc-attR containing the ter_*thrL* transcription terminator and attR.
4. The required artificial DNA fragment (1156 bp) was obtained by PCR amplification of the ligation reaction mixture using oligonucleotides P9 and P4 (SEQ ID NOS: 22 and 16) as primers (these primers contained the subsidiary recognition sites for *Hind*III and *Xba*I endonucleases).

### (2) pMW-intxis-ts

Recombinant plasmid pMW-intxis-ts containing the *cI* repressor gene and the *int-xis* genes of phage λ under control of promoter P_{R} was constructed on the basis of vector pMWP_{lac}lacI-ts. To construct the pMWP_{lac}lacI-ts variant, the *Aat*II*-Eco*RV fragment of the pMWP_{lac}lacI plasmid (Skorokhodova, A. Yu. et al., Biotekhnologiya (in Russian), 2004, no. 5, 3-21) was substituted with the *Aat*II*-Eco*RV fragment of the pMAN997 plasmid (Tanaka, K. et al., J. Bacteriol., 2001, 183(22): 6538-6542, WO99/03988) bearing the *par* and *ori* loci and the *repA*^{ts} gene (a temperature sensitive-replication origin) of the pSC101 replicon. The plasmid pMAN997 was constructed by exchanging the VspI-HindIII fragments of pMAN031 (J. Bacteriol., 162, 1196 (1985)) and pUC19.

Two DNA fragments were amplified using phage λ DNA ("Fermentas") as a template. The first one contained the DNA sequence from 37168 to 38046, the *cI* repressor gene, promoters P_{RM} and P_{R}, and the leader sequence of the *cro* gene. This fragment was PCR-amplified using oligonucleotides P13 and P14 (SEQ ID NOS: 27 and 28) as primers. The second DNA fragment containing the *xis-int* genes of phage λ and the DNA sequence from 27801 to 29100 was PCR-amplified using oligonucleotides P15 and P16 (SEQ ID NOS: 29 and 30) as primers. All primers contained the corresponding restriction sites.

The first PCR-amplified fragment carrying the *cI* repressor was digested with restriction endonuclease *Cla*I, treated with Klenow fragment of DNA polymerase I, and then digested with restriction endonuclease *Eco*RI*.* The second PCR-amplified fragment was digested with restriction endonucleases *Eco*RI and *Pst*I. The pMWP_{lac}lacI-ts plasmid was digested with the *Bgl*II endonuclease, treated with Klenow fragment of DNA polymerase I, and digested with the *Pst*I restriction endonuclease. The vector fragment of pMWPlaclacI-ts was eluted from agarose gel and ligated with the above-mentioned digested PCR-amplified fragments to obtain the pMW-intxis-ts recombinant plasmid.

### Example 2. Replacement of the native promoter regions for the fucPIKUR operon in E. coli with the hybrid P_{L-tac} promote

To replace the native promoter region of the *fucPICUR* operon with a more potent promoter, the DNA fragment carrying the hybrid P_{L-tac} promoter and the chloramphenicol resistance marker (Cm^{R}) encoded by the *cat* gene was integrated into the chromosome of *E. coli* MG1655 (ATCC 700926) instead of the native promoter region by the method described by Datsenko K.A. and Wanner B.L. (Proc. Natl. Acad. Sci. USA, 2000, 97: 6640-6645) called "Red-mediated integration" and/or "Red-driven integration".. The pKD46 recombinant plasmid (Datsenko, K.A. and Wanner, B.L., Proc. Natl. Acad. Sci. USA, 2000, 97: 6640-6645) with the thermosensitive replicon was used as a donor of the phage λ-derived genes responsible for the Red-mediated recombination system. The *E. coli* BW25113 containing the pKD46 recombinant plasmid can be obtained from the *E*. *coli* Genetic Stock Center, Yale University, New Haven, U.S.A. (accession number CGSC7630).

The hybrid P_{L-tac} promoter was synthesized chemically (SEQ ID NO: 31). The synthesized DNA fragment containing the hybrid P_{L-tac} promoter bears the *Bgl*II recognition site at the 5' end, which is necessary to further join the *cat* gene, and a 36-bp region complementary to the 5' end of the *fucPIKUR* operon required for further integration into the bacterial chromosome.

The DNA fragment containing the Cm^{R} marker encoded by the *cat* gene was obtained by PCR, using primers P17 (SEQ ID NO: 32) and P18 (SEQ ID NO: 33) and plasmid pMW118-attL-Cm-attR as a template (for construction see Example 1). Primer P17 contains a 36-bp region complementary to the DNA region located 190 bp upstream of the start codon of the *fucPIKUR* operon. Primer P18 contains the *Bgl*II recognition site at the 5' end required for further joining the hybrid P_{L-tac} promoter.

PCR was conducted using a ThermoHybaid PCR Express amplificator (Thermo Electron Corporation). The reaction mixture (in total volume of 50 µl) included 5 µl of PCR buffer (tenfold) containing 15 mM MgCl₂ ("Fermentas", Lithuania), 200 µM each of dNTP, 25 pmol each of the exploited primers, and 1 U of Taq-polymerase ("Fermentas", Lithuania). Approximately 5 ng of the plasmid DNA was added to the reaction mixture as a template. The following temperature profile was used: the initial DNA denaturation at 95°C for 5 min followed by 25 cycles (denaturation at 95°C for 30 s, annealing at 55°C for 30 s, elongation at 72°C for 30 s) and the final elongation at 72°C for 7 min. Then the amplified DNA fragment was purified by electrophoresis in agarose gel, extracted using "GenElute Spin Columns" ("Sigma", USA), and precipitated by ethanol.

The DNA fragment containing the hybrid P_{L-tac} promoter and the DNA fragment containing the Cm^{R} marker were treated with *Bgl*II and ligated. The ligation product was amplified by PCR using primers P17 (SEQ ID NO: 32) and P19 (SEQ ID NO: 34). Primer P19 contains a 36-bp region at the 5' end that is complementary to the 5' end of the *fucPIKUR* operon and is required for further integration into the bacterial chromosome.

The amplified DNA fragment was purified by electrophoresis in agarose gel, extracted using "GenElute Spin Columns" ("Sigma", USA), and precipitated by ethanol. The obtained DNA fragment was used for electroporation and Red-mediated integration into the *E. coli* MG1655/pKD46 chromosome.

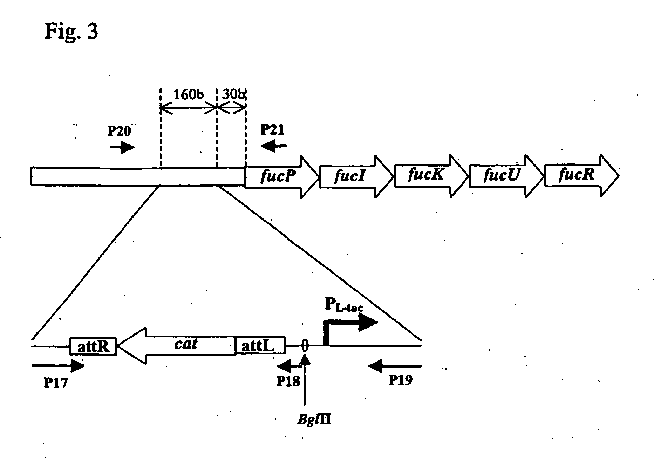

The *E. coli* MG1655/pKD46 was grown at 30°C overnight in liquid LB medium supplemented with ampicillin (100 µg/ml). Then the culture was 100-fold diluted with SOB medium containing yeast extract (5 g/l), NaCl (0.5 g/l), tryptone (20 g/l), KCl (2.5 mM), MgCl₂ (10 mM) supplemented with ampicillin (100 µg/ml) and L-arabinose (10 mM) [arabinose was used to induce the plasmid encoding the Red system genes] and was grown at 30°C to reach the optical density of OD₆₀₀=0.4-0.7. The cells collected from 10 ml of the bacterial culture were washed three times with ice-cold deionized water and then suspended in 100 µl of the water. The DNA fragment (10 µl, 100 ng) dissolved in deionized water was added to the cell suspension. The electroporation was done using a "BioRad" electroporator (USA, No. 165-2098, version 2-89) according to the manufacturer's instructions. The shocked cells were diluted with 1 ml of SOC medium (Sambrook et al, "Molecular Cloning. A Laboratory Manual, Second Edition", Cold Spring Harbor Laboratory Press, 1989), incubated at 37°C for 2 h, and then spread on L-agar containing chloramphenicol (25 µg/ml). After 24 h of growth, colonies were tested for the presence of Cm^{R} marker by PCR using primers P20 (SEQ ID NO: 35) and P21 (SEQ ID NO: 36). For this purpose, a freshly isolated colony was suspended in water (20µl) and 1µl of the obtained suspension was used for PCR. The temperature profile was as follows: the initial DNA denaturation at 95°C for 10 min; then 30 cycles (denaturation at 95°C for 30 s, annealing at 55°C for 30 s, and elongation at 72°C for 1 min), and a final elongation at 72°C for 7 min. A few Cm^{R} colonies tested contained the required ~2000-bp DNA fragment, confirming the substitution of the 190-bp native promoter region of the *fucPIKUR* operon by the hybrid P_{L-tac} promoter (see Figure 3). One of the obtained strains was cured from the thermosensitive plasmid pKD46 by culturing at 37°C and the resulting strain was named *E*. *coli* MG1655P_{L-tac}fucPIKUR.

### Example 3. Effect of enhanced expression of the fucPIKUR operon on growth of the E. coli strain having the PTS transport system disrupted

To demonstrate the effect of enhanced expression of the *fucPIKUR* operon on cell growth, the *E*. *coli* strain having a disrupted PTS (phosphoenolpyruvate-sugar transport system) was constructed.

The DNA fragment carrying the kanamycin resistance marker (Km^{R}) was integrated into the chromosome of *E. coli* MG1655/pKD46 instead of the *ptsHI-crr* operon by the method described by Datsenko K.A. and Wanner B.L. (Proc. Natl. Acad. Sci. USA, 2000, 97, 6640-6645) called "Red-mediated integration" and/or "Red-driven integration" as described in Example 2.

The *ptsHI-crr* operon has been elucidated (nucleotide positions: 2531786 to 2532043, 2532088 to 2533815, and 2533856 to 2534365 for *ptsH, ptsI,* and *crr* genes, respectively; GenBank accession no. NC_000913.2; gi: 49175990). The *ptsHI-crr* operon is located between *cysK* and *pdxK* genes on the *E. coli* K-12 chromosome.

The DNA fragment carrying the Km^{R} gene was obtained by PCR using the commercially available plasmid pUC4KAN (GenBank/EMBL accession no. X06404; "Fermentas", Lithuania) as the template and primers P22 (SEQ ID NO: 37) and P23 (SEQ ID NO: 38). Primer P22 contained a 36-nt sequence complementary to the 5' end of the *ptsH* gene and primer P23 contained a 36-nt sequence complementary to the 3'end of the *crr* gene. These nucleotide sequences were introduced into primers P22 and P23 for further integration into the bacterial chromosome.

PCR was conducted as described in Example 2.

The PCR-amplified DNA fragment was purified by agarose gel-electrophoresis, extracted from the gel by centrifugation through a "GenElute Spin Column" ("Sigma", USA), and precipitated by ethanol. The obtained DNA fragment was used for electroporation and Red-mediated integration into the chromosome of *E. coli* MG1655/pKD46 as described in Example 2, except that after electroporation, cells were spread onto L-agar containing 50 µg/ml of kanamycin.

After 24 h of growth, the bacterial colonies were tested for the presence of the Km^{R} marker instead of *ptsHI-crr* operon by PCR using primers P24 (SEQ ID NO: 39) and P25 (SEQ ID NO: 40). For this purpose, a freshly isolated colony was suspended in 20µl of water, and 1µl of the resulting suspension was used for PCR. The PCR conditions were described in Example 2. A few Km^{R} colonies tested contained the required ~1300 bp DNA fragment, which confirmed the presence of the Km^{R} gene instead of the *ptsHI-crr* operon. One of the strains obtained was cured from thermosensitive plasmid pKD46 by culturing at 37°C, and the resulting strain was named *E. coli* MG1655 Δ*ptsHI-crr.*

The DNA fragment from the chromosome of the above-mentioned *E*. *coli* MG1655P_{L-tac}fucPIKUR was transferred to *E. coli* MG1655 Δ*ptsHI-crr* by P1 transduction (Miller, J.H. (1972) Experiments in Molecular Genetics, Cold Spring Harbor Lab. Press, Plainview, NY), resulting in strain MG1655 Δ*ptsHI-crr* P_{L-tac}fucPIKUR.

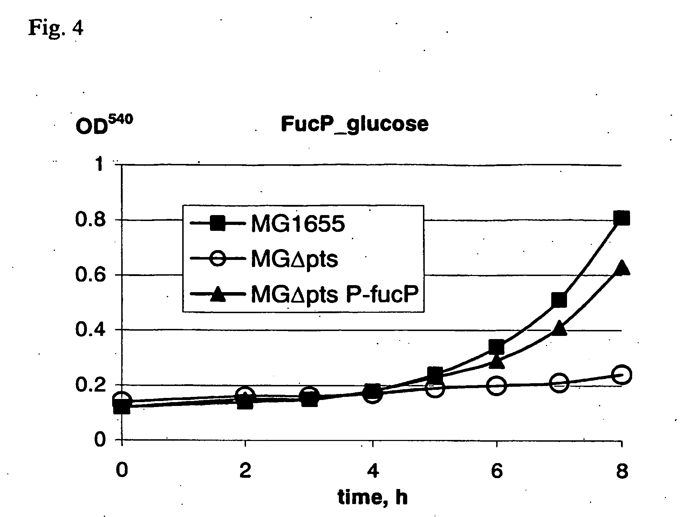

The ability to grow on minimal medium with glucose (4%) as a carbon source was verified for the three following *E. coli* strains: MG1655, MG1655 Δ*ptsHI-crr,* and MG1655 Δ*ptsHI-crr* P_{L-tac}fucPIKUR (Fig. 4). As follows from Fig. 4, *E*. *coli* MG1655 Δ*ptsHI-crr* P_{L-tac}fucPIKUR demonstrated a substantially higher growth rate, as compared with MG1655 Δ*ptsHI-crr,* indicating the fact that enhancing expression of the *fucPIKUR* operon significantly increased the growth characteristics of the recipient strain on minimal medium containing glucose.

### Example 4. Production of L-threonine by E. coli B-3996P_{L-tac}fucPIKUR

To test the effect of enhanced expression of the *fucPIKUR* operon (under the P_{L-*tac*} promoter control) on threonine production, DNA fragments from the chromosome of the above-described *E. coli* strain MG1655P_{L-tac}fuPIKUR were transferred to the threonine-producing *E. coli* strain B-3996 (VKPM B-3996) by P1 transduction (Miller, J.H. Experiments in Molecular Genetics, Cold Spring Harbor Lab. Press, 1972, Plainview, NY).

Both *E. coli* strains, B-3996 and B-3996P_{L-tac}fucPIKUR, were grown for 18-24 hours at 37°C on L-agar plates. To obtain a seed culture, the strains were grown on a rotary shaker (250 rpm) at 32°C for 18 hours in 20x200-mm test tubes containing 2 ml of L-broth supplemented with 4% glucose. Then, the fermentation medium was inoculated with 0.21 ml (10%) seed material. The fermentation was performed in 2 ml of minimal medium for fermentation in 20x200-mm test tubes. Cells were grown for 72 hours at 32°C with shaking at 250 rpm.

After cultivation, the amount of L-threonine, which had accumulated in the medium, was determined by paper chromatography using the following mobile phase: butanol-acetic acid-water, 4:1:1 (v/v). A solution of ninhydrin (2%) in acetone was used as a visualizing reagent. A spot containing L-threonine was cut out, L-threonine was eluted with 0.5% water solution of CdCl₂, and the amount of L-threonine was estimated spectrophotometrically at 540 nm. The results of four independent test tube fermentations are shown in Table 1.

The composition of the fermentation medium (g/l) was as follows:

| | |
|---|---|
| Glucose | 80.0 |
| (NH₄)₂SO₄ | 22.0 |
| NaCl | 0.8 |
| KH₂PO₄ | 2.0 |
| MgSO₄·7H₂O | 0.8 |
| FeSO₄·7H₂O | 0.02 |
| MnSO₄·5H₂O | 0.02 |
| Thiamine HCl | 0.0002 |
| Yeast extract | 1.0 |
| CaCO₃ | 30.0 |

Glucose and magnesium sulfate were sterilized separately. CaCO₃ was sterilized by dry-heat at 180°C for 2 hours. The pH was adjusted to 7.0. The antibiotic was introduced into the medium after sterilization.

**Table 1**

| Strain | 48 h | | 72 h | |
|---|---|---|---|---|
| | OD₅₄₀ | L-threonine, g/l | OD₅₄₀ | L-threonine, g/l |
| B-3996 | 19.1±0.3 | 16.2±0.5 | 17.1±0.3 | 24.8±0.3 |
| B-3996P_{L-tac}fucPIKUR | 18.9±0.2 | 18.0±0.4 | 17.9±0.3 | 29.2±0.6 |

As follows from Table 1, B-3996P_{L-tac}fucPIKUR accumulated a higher amount of L-threonine, as compared with B-3996.

### Example 5. Production of L-lysine by E. coli WC196(pCABD2)(pCaBD2)_{L-tac}fucPIKUR

To test the effect of enhanced expression of the *fucPIKUR* operon (under the P_{L-*tac*} promoter control) on lysine production, DNA fragments from the chromosome of the above-described *E. coli* strain MG1655P_{L-tac}fucPIKUR can be transferred to the lysine-producing *E. coli* strain WC196 (pCABD2) by P1 transduction (Miller, J.H. Experiments in Molecular Genetics, Cold Spring Harbor Lab. Press, 1972, Plainview, NY). The pCABD2 plasmid includes the *dapA* gene encoding dihydrodipicolinate synthase having a mutation which desensitizes the feedback inhibition by L-lysine, the *lysC* gene encoding aspartokinase III having a mutation which desensitizes the feedback inhibition by L-lysine, the *dapB* gene encoding dihydrodipicolinate reductase, and the *ddh* gene encoding diaminopimelate dehydrogenase (U.S. Patent No. 6,040,160).

Both *E. coli* strains, WC196(pCABD2) and WC196(pCABD2)P_{L-tac}fucPIKUR, can be cultured in Medium containing streptomycin (20 mg/l) at 37°C, and 0.3 ml of the obtained culture can be inoculated into 20 ml of the fermentation medium containing the required drugs in a 500-ml flask. The cultivation can be carried out at 37°C for 16 h by using a reciprocal shaker at the agitation speed of 115 rpm. After the cultivation, the amounts of L-lysine and residual glucose in the medium can be measured by a known method (Biotech-analyzer AS210 manufactured by Sakura Seiki Co.). Then, the yield of L-lysine can be calculated on consumed glucose for each of the strains.

The composition of fermentation medium (g/l) is as follows:

| | |
|---|---|
| Glucose | 40 |
| (NH₄)₂SO₄ | 24 |
| K₂HPO₄ | 1.0 |
| MgSO₄·7H₂O | 1.0 |
| FeSO₄·7H₂O | 0.01 |
| MnSO₄·5H₂O | 0.01 |
| Yeast extract | 2.0 |

The pH is adjusted to 7.0 by KOH and the medium is autoclaved at 115°C for 10 min. Glucose and MgSO₄·7H₂O are sterilized separately. CaCO₃ is dry-heat sterilized at 180°C for 2 hours and added to the medium for a final concentration of 30 g/l.

### Example 6. Production of L-cysteine by E. coli JM15(ydeD)P_{L-tac}fucPIKUR

To test the effect of enhanced expression of the *fucPIKUR* operon (under the P_{L-*tac*} promoter control) on L-cysteine production, DNA fragments from the chromosome of the above-described *E. coli* strain MG1655P_{L-tac}fucPIKUR can be transferred to the L-cysteine-producing *E. coli* strain JM15(ydeD) by P1 transduction (Miller, J.H. Experiments in Molecular Genetics, Cold Spring Harbor Lab. Press, 1972, Plainview, NY) to obtain the strain JM15(ydeD)P_{L-tac}fucPIKUR.

*E. coli* JM15(ydeD) is a derivative of *E. coli* JM15 (U.S. Patent No. 6,218,168), which can be transformed with DNA having the *ydeD* gene encoding a membrane protein, and is not involved in a biosynthetic pathway of any L-amino acid (U.S. Patent No. 5,972,663). The strain JM15 (CGSC# 5042) can be obtained from The Coli Genetic Stock Collection at the *E. coli* Genetic Resource Center, MCD Biology Department, Yale University (htt.p://cgsc.biology.yale.edu/).

Fermentation conditions for evaluation of L-cysteine production were described in detail in Example 6 of U.S. Patent No. 6,218,168.

### Example 7. Production of L-leucine by E. coli 57P_{L-tac}fucPIKUR

To test the effect of enhanced expression of the *fucPIKUR* operon (under the P_{L-tac} promoter control) on L-leucine production, DNA fragments from the chromosome of the above-described *E. coli* strain MG1655P_{L-tac}fucPIKUR can be transferred to the L-leucine-producing *E*. *coli* strain 57 (VKPM B-7386, U.S. Patent No. 6,124,121) by P1 transduction (Miller, J.H. Experiments in Molecular Genetics, Cold Spring Harbor Lab. Press, 1972, Plainview, NY) to obtain the strain 57-pMW-ΔfucPIKUR. The strain 57 has been deposited in the Russian National Collection of Industrial Microorganisms (VKPM) (Russia, 117545 Moscow, 1 Dorozhny proezd, 1) on May 19, 1997 under accession number VKPM B-7386.

Both *E. coli* strains, 57 and 57P_{L-tac}fucPIKUR, can be cultured for 18-24 hours at 37°C on L-agar plates. To obtain a seed culture, the strains can be grown on a rotary shaker (250 rpm) at 32°C for 18 hours in 20x200-mm test tubes containing 2 ml of L-broth supplemented with 4% glucose. Then, the fermentation medium can be inoculated with 0.21 ml of seed material (10%). The fermentation can be performed in 2 ml of a minimal fermentation medium in 20x200-mm test tubes. Cells can be grown for 48-72 hours at 32°C with shaking at 250 rpm. The amount of L-leucine can be measured by paper chromatography (liquid phase composition: butanol - acetic acid - water = 4:1:1)

The composition of the fermentation medium (g/l) is as follows (pH 7.2):

| | |
|---|---|
| Glucose | 60.0 |
| (NH₄)₂SO₄ | 25.0 |
| K₂HPO₄ | 2.0 |
| MgSO₄·7H₂O | 1.0 |
| Thiamine | 0.01 |
| CaCO₃ | 25.0 |

Glucose and CaCO₃ are sterilized separately.

### Example 8. Production of L-histidine by E. coli 80P_{L-tac}fucPIKUR

To test the effect of enhanced expression of the *fucPIKUR* operon (under the P_{L-*tac*} promoter control) on L-histidine production, DNA fragments from the chromosome of the above-described *E. coli* strain MG1655P_{L-tac}fucPIKUR can be transferred to the histidine-producing *E. coli* strain 80 by P1 transduction (Miller, J.H. Experiments in Molecular Genetics, Cold Spring Harbor Lab. Press, 1972, Plainview, NY). The strain 80 has been described in Russian patent 2119536 and deposited in the Russian National Collection of Industrial Microorganisms (Russia, 117545 Moscow, 1 Dorozhny proezd, 1) on October 15, 1999 under accession number VKPM B-7270 and then converted to a deposit under the Budapest Treaty on July 12, 2004.

Both *E*. *coli* strains, 80 and 80P_{L-tac}fucPIKUR, can be cultured in L-broth for 6 hours at 29°C. Then, 0.1 ml of obtained culture can be inoculated into 2 ml of fermentation medium in a 20x200-mm test tube and cultivated for 65 hours at 29°C with shaking on a rotary shaker (350 rpm). After cultivation, the amount of histidine which accumulates in the medium can be determined by paper chromatography. The paper can be developed with a mobile phase consisting of n-butanol: acetic acid: water = 4: 1: 1 (v/v). A solution of ninhydrin (0.5%) in acetone can be used as a visualizing reagent.

The composition of the fermentation medium (pH 6.0) is as follows (g/l):

| | |
|---|---|
| Glucose | 100.0 |
| Mameno (soybean hydrolysate) | 0.2 as total nitrogen |
| L-proline | 1.0 |
| (NH₄)₂SO₄ | 25.0 |
| KH₂PO₄ | 2.0 |
| MgSO₄·7H₂0 | 1.0 |
| FeSO4·7H₂0 | 0.01 |
| MnSO₄ | 0.01 |
| Thiamine | 0.001 |
| Betaine | 2.0 |
| CaCO₃ | 60.0 |

Glucose, proline, betaine and CaCO₃ are sterilized separately. The pH is adjusted to 6.0 before sterilization.

### Example 9. Production of L-glutamate by E. coli VL334thrC⁺P_{L-tac}fucPIKUR

To test the effect of enhanced expression of the *fucPIKUR* operon (under the P_{L-*tac*} promoter control) on L-glutamate production, DNA fragments from the chromosome of the above-described *E. coli* strain MG1655P_{L-tac}fucPIKUR can be transferred to the L-glutamate-producing *E*. *coli* strain VL334thrC⁺ (EP 1172433) by P1 transduction (Miller, J.H. Experiments in Molecular Genetics, Cold Spring Harbor Lab. Press, 1972, Plainview, NY) to obtain the strain VL334thrC⁺P_{L-tac}fucPIKUR. The strain VL334thrC⁺ has been deposited in the Russian National Collection of Industrial Microorganisms (VKPM) (Russia, 117545 Moscow, 1 Dorozhny proezd, 1) on December 6, 2004 under the accession number VKPM B-8961 and then converted to a deposit under the Budapest Treaty on December 8, 2004.

Both *E. coli* strains, VL334thrC⁺ and VL334thrC⁺P_{L-tac}fucPIKUR, can be grown for 18-24 hours at 37°C on L-agar plates. Then, one loop of the cells can be transferred into test tubes containing 2ml of fermentation medium. The fermentation medium contains glucose (60g/l), ammonium sulfate (25 g/l), KH₂PO₄ (2g/l), MgSO₄ (1 g/l), thiamine (0.1 mg/ml), L-isoleucine (70 µg/ml), and CaCO₃ (25 g/l). The pH is adjusted to 7.2. Glucose and CaCO₃ are sterilized separately. Cultivation can be carried out at 30°C for 3 days with shaking. After the cultivation, the amount of L-glutamic acid produced can be determined by paper chromatography (liquid phase composition of butanol-acetic acid-water=4:1:1) with subsequent staining by ninhydrin (1% solution in acetone) and further elution of the compounds in 50% ethanol with 0.5% CdCl₂.

### Example 10. Production of L-phenylalanine by E. coli AJ12739P_{L-tac}fucPIKUR

To test the effect of enhanced expression of the *fucPIKUR* operon (under the P_{L-*tac*} promoter control) on L-phenylalanine production, DNA fragments from the chromosome of the above-described *E. coli* strain MG1655P_{L-tac}fucPIKUR can be transferred to the phenylalanine-producing *E. coli* strain AJ12739 by P1 transduction (Miller, J.H. Experiments in Molecular Genetics, Cold Spring Harbor Lab. Press, 1972, Plainview, NY). The strain AJ12739 has been deposited in the Russian National Collection of Industrial Microorganisms (VKPM) (Russia, 117545 Moscow, 1 Dorozhny proezd, 1) on November 6, 2001 under accession number VKPM B-8197 and then converted to a deposit under the Budapest Treaty on August 23, 2002.

Both *E. coli* strains, AJ12739 and AJ12739P_{L-tac}fucPIKUR, can each be cultivated at 37°C for 18 hours in a nutrient broth, and 0.3 ml of the obtained culture can be inoculated into 3 ml of a fermentation medium in a 20x200-mm test tube and cultivated at 37°C for 48 hours with shaking on a rotary shaker. After cultivation, the amount of phenylalanine which accumulatesin the medium can be determined by TLC. The 10x15-cm TLC plates coated with 0.11-mm layers of Sorbfil silica gel containing no fluorescent indicator (Stock Company Sorbpolymer, Krasnodar, Russia) can be used. The Sorbfil plates can be developed with a mobile phase consisting of propan-2-ol-ethyl acetate: 25% aqueous ammonia: water = 40: 40: 7: 16 (v/v). A solution of ninhydrin (2%) in acetone can be used as a visualizing reagent.

The composition of the fermentation medium (g/l) is as follows:

| | |
|---|---|
| Glucose | 40.0 |
| (NH₄)₂SO₄ | 16.0 |
| K₂HPO₄ | 0.1 |
| MgSO₄·7H₂O | 1.0 |
| FeSO₄·7H₂O | 0.01 |
| MnSO₄·5H₂O | 0.01 |
| Thiamine HCl | 0.0002 |
| Yeast extract | 2.0 |
| Tyrosine | 0.125 |
| CaCO₃ | 20.0 |

Glucose and magnesium sulfate are sterilized separately. CaCO₃ is dry-heat sterilized at 180° for 2 hours. The pH is adjusted to 7.0.

### Example 11. Production of L-tryptophan by E. coli SV164 (pGH5)P_{L-tac}fucPIKUR

To test the effect of enhanced expression of the *fucPIKUR* operon (under the P_{L-*tac*} promoter control) on L-tryptophan production, DNA fragments from the chromosome of the above-described *E*. *coli* strain MG1655P_{L-tac}fucPIKUR can be transferred to the tryptophan-producing *E*. *coli* strain SV164 (pGH5) by P1 transduction (Miller, J.H. Experiments in Molecular Genetics, Cold Spring Harbor Lab. Press, 1972, Plainview, NY). The strain SV164 has the *trpE* allele encoding anthranilate synthase free from feedback inhibition by tryptophan. The plasmid pGH5 harbors a mutant *serA* gene encoding phosphoglycerate dehydrogenase free from feedback inhibition by serine.The strain SV164 (pGH5) is described in detail in U.S. Patent No. 6,180,373.

Both *E*. *coli* strains, SV164(pGH5) and SV164(pGH5)P_{L-tac}fucPIKUR, can be cultivated with shaking at 37°C for 18 hours in 3 ml of nutrient broth supplemented with tetracycline (20 mg/l, marker of pGH5 plasmid). The obtained cultures (0.3 ml each) can be each inoculated into 3 ml of a fermentation medium containing tetracycline (20 mg/l) in 20 x 200-mm test tubes, and cultivated at 37°C for 48 hours with a rotary shaker at 250 rpm. After cultivation, the amount of tryptophan which accumulates in the medium can be determined by TLC as described in Example 10. The fermentation medium components are listed in Table 2, and are sterilized in separate groups (A, B, C, D, E, F, and H), as shown, to avoid adverse interactions during sterilization.

**Table 2**

| Groups | Component | Final concentration, g/l |
|---|---|---|
| A | KH₂PO₄ | 1.5 |
| | NaCl | 0.5 |
| | (NH₄)₂SO₄ | 1.5 |
| | L-Methionine | 0.05 |
| | L-Phenylalanine | 0.1 |
| | L-Tyrosine | 0.1 |
| | Mameno (total N) | 0,07 |
| B | Glucose | 40.0 |
| | MgSO₄·7H₂O | 0.3 |
| C | CaCl₂ | 0.011 |
| D | FeSO₄·7H₂O | 0.075 |
| | Sodium citrate | 1.0 |
| E | Na₂MoO₄·2H₂O | 0.00015 |
| | H₃BO₃ | 0.0025 |
| | CoCl₂·6H₂O | 0.00007 |
| | CuSO₄·5H₂O | 0.00025 |
| | MnCl₂·4H₂O | 0.0016 |
| | ZnSO₄·7 H₂O | 0.0003 |
| F | Thiamine HCl | 0.005 |
| G | CaCO₃ | 30.0 |
| H | Pyridoxine | 0.03 |

Group A has pH 7.1 adjusted by NH₄OH. Each of groups A, B, C, D, E, F and H is sterilized separately, chilled, and mixed together, and then CaCO₃ sterilized by dry heat is added to the complete fermentation medium.

### Example 12. Production of L-proline by E. coli 702ilvAP_{L-tac}fucPIKUR

To test the effect of enhanced expression of the *fucPIKUR* operon (under the P_{L-*tac*} promoter control) on L-proline production, DNA fragments from the chromosome of the above-described *E. coli* strain MG1655P_{L-tac}fucPIKUR can be transferred to the proline-producing *E. coli* strain 702ilvA by P1 transduction (Miller, J.H. Experiments in Molecular Genetics, Cold Spring Harbor Lab. Press, 1972, Plainview, NY). The strain 702ilvA has been deposited in the Russian National Collection of Industrial Microorganisms (VKPM) (Russia, 117545 Moscow, 1 Dorozhny proezd, 1) on July 18,2000 under accession number VKPM B-8012 and then converted to a deposit under the Budapest Treaty on May 18, 2001.

Both *E. coli* strains, 702ilvA and 702ilvAP_{L-tac}fucPIKUR, can be grown for 18-24 hours at 37°C on L-agar plates. Then, these strains can be cultivated under the same conditions as in Example 9.

### Example 13. Production of L-arginine by E. coli 382P_{L-tac}fucPIKUR

To test the effect of enhanced expression of the *fucPIKUR* operon (under the P_{L-*tac*} promoter control) on L-arginine production, DNA fragments from the chromosome of the above-described *E. coli* strain MG1655P_{L-tac}fucPIKUR can be transferred to the arginine-producing *E. coli* strain 382 by P1 transduction (Miller, J.H. Experiments in Molecular Genetics, Cold Spring Harbor Lab. Press, 1972, Plainview, NY). The strain 382 has been deposited in the Russian National Collection of Industrial Microorganisms (VKPM) (Russia, 117545 Moscow, 1 Dorozhny proezd, 1) on April 10, 2000 under accession number VKPM B-7926 and then converted to a deposit under the Budapest Treaty on May 18, 2001.

Both strains, 382 and 382P_{L-tac}fucPIKUR, can be cultivated with shaking at 37°C for 18 hours in 3 ml of nutrient broth. The obtained cultures (0.3 ml each) can be inoculated into 3 ml of a fermentation medium in 20 x 200-mm test tubes and cultivated at 32°C for 48 hours on a rotary shaker.

After the cultivation, the amount of L-arginine which accumulates in the medium can be determined by paper chromatography using the following mobile phase: butanol: acetic acid: water = 4: 1: 1 (v/v). A solution of ninhydrin (2%) in acetone can be used as a visualizing reagent. A spot containing L-arginine can be cut out, L-arginine can be eluted with 0.5% water solution of CdCl₂, and the amount of L-arginine can be estimated spectrophotometrically at 540 nm.

The composition of the fermentation medium (g/l) is as follows:

| | |
|---|---|
| Glucose | 48.0 |
| (NH4)₂SO₄ | 35.0 |
| KH₂PO₄ | 2.0 |
| MgSO₄·7H₂O | 1.0 |
| Thiamine HCl | 0.0002 |
| Yeast extract | 1.0 |
| L-isoleucine | 0.1 |
| CaCO3 | 5.0 |

Glucose and magnesium sulfate are sterilized separately. CaCO₃ is dry-heat sterilized at 180 °C for 2 hours. The pH is adjusted to 7.0.

### Example 14. Elimination of Cm resistance gene (cat gene) from the chromosome of L-amino acid producing E. coli strains.

Cm resistance gene (*cat* gene) can be eliminated from the chromosome of the L-amino acid producing strain using *int-xis* system. For that purpose, L-amino acid producing strains, in which DNA fragments from the chromosome of the above-described *E. coli* strain MG1655P_{L-tac}fucPIKUR was transferred by P1 transduction (see Examples 4-13), can be transformed with plasmid pMWts-Int/Xis. Transformant clones can be selected on the LB-medium containing 100 µg/ml of ampicillin. Plates can be incubated overnight at 30°C. Transformant clones can be cured from *cat* gene by spreading the separate colonies at 37°C (at that temperature repressor CIts is partially inactivated and transcription of *int*/*xis* genes is derepressed) followed by selection of Cm^{S}Ap^{R} variants. Elimination of the *cat* gene from the chromosome of the strain can be verified by PCR. Locus-specific primers P20 (SEQ ID NO: 35) and P21 (SEQ ID NO: 36) can be used in PCR for the verification. Conditions for PCR verification can be as described above. The PCR product obtained in the reaction with the cells with eliminated *cat* gene as a template, should be 0,2 kbp in length. Thus, the L-amino acid producing strain with enhanced expression of *fucPIKUR* operon and eliminated *cat* gene can be obtained.

### Industrial Applicability

According to the present invention, production of L-amino acid of a bacterium of the *Enterobacteriaceae* family can be enhanced.

## Claims

1. A method for producing an L-amino acid comprising: cultivating an L-amino acid-producing bacterium of the Enterobacteriaceae family, wherein said bacterium has been modified to enhance expression of at least one gene of the fucPIKUR operon in a medium to produce and excrete said L-amino acid into the medium, and collecting said L-amino acid from the medium.

2. The method according to claim 1, wherein said gene expression is enhanced by modifying an expression control sequence for the fucPIKUR operon or by increasing the copy number for at least one gene of the fucPIKUR operon.

3. The method according to claim 1 or 2, wherein said bacterium belongs to the genus Escherichia.

4. The method according to claim 3, wherein said bacterium belongs to Escherichia coli.

5. The method according to claim 1 or 2, wherein said bacterium belongs to the genus Pantoea.

6. The method according to any of claims 1 to 5, wherein said L-amino acid is selected from the group consisting of an aromatic L-amino acid and a non-aromatic L-amino acid.

7. The method according to claim 6, wherein said aromatic L- amino acid is selected from the group consisting of L-phenylalanine, L-tyrosine, and L-tryptophan.

8. The method according to claim 6, wherein said non-aromatic L-amino acid is selected from the group consisting of L-threonine, L-lysine, L-cysteine, L-methionine, L-leucine, L-isoleucine, L-valine, L-histidine, glycine, L-serine, L- alanine, L-asparagine, L-aspartic acid, L-glutamine, L-glutamic acid, L-proline, and L- arginine.

9. The method according to claim 8, wherein said non-aromatic L-amino acid is L-threonine.

## Patentansprüche

1. Verfahren zur Herstellung einer L-Aminosäure, umfassend: Kultivieren eines L-Aminosäureproduzierenden Bakteriums aus der Enterobacteriaceae-Familie in einem Medium, wobei das Bakterium verändert wurde, um die Expression von wenigstens einem Gen des fucPIKUR-Operons zu verstärken, um die L-Aminosäure herzustellen und in das Medium auszuscheiden, und Sammeln der L-Aminosäure aus dem Medium.

2. Verfahren nach Anspruch 1, wobei die Genexpression durch Veränderung einer Expressionskontrollsequenz für das fucPIKUR-Operon oder durch Erhöhung der Kopienzahl von wenigstens einem Gen des fucPIKUR-Operons verstärkt wird.

3. Verfahren nach Anspruch 1 oder 2, wobei das Bakterium zur Gattung Escherichia gehört.

4. Verfahren nach Anspruch 3, wobei das Bakterium zu Escherichia coli gehört.

5. Verfahren nach Anspruch 1 oder 2, wobei das Bakterium zur Gattung Pantoea gehört.

6. Verfahren nach einem der Ansprüche 1 bis 5, wobei die L-Aminosäure aus der Gruppe bestehend aus einer aromatischen L-Aminosäure und einer nichtaromatischen L-Aminosäure ausgewählt wird.

7. Verfahren nach Anspruch 6, wobei die aromatische L-Aminosäure aus der Gruppe bestehend aus L-Phenylalanin, L-Tyrosin und L-Tryptophan ausgewählt wird.

8. Verfahren nach Anspruch 6, wobei die nichtaromatische L-Aminosäure aus der Gruppe bestehend aus L-Threonin, L-Lysin, L-Cystein, L-Methionin, L-Leucin, L-Isoleucin, L-Valin, L-Histidin, Glycin, L-Serin, L-Alanin, L-Asparagin, L-Asparaginsäure, L-Glutamin, L-Glutaminsäure, L-Prolin und L-Arginin ausgewählt wird.

9. Verfahren nach Anspruch 8, wobei die nichtaromatische L-Aminosäure L-Threonin ist.

## Revendications

1. Procédé pour produire un acide L-aminé comprenant :
la culture d'une bactérie de production d'acide L-aminé de la famille des Enterobacteriaceae, dans lequel ladite bactérie a été modifiée pour améliorer l'expression d'au moins un gène de l'opéron fucPIKUR dans un milieu pour produire et excréter ledit acide L-aminé dans le milieu, et la collecte dudit acide L-aminé à partir du milieu.

2. Procédé selon la revendication 1, dans lequel ladite expression de gène est améliorée en modifiant une séquence de commande d'expression pour l'opéron fucPIKUR ou en augmentant le nombre de copie pour au moins un gène de l'opéron fucPIKUR.

3. Procédé selon la revendication 1 ou 2, dans lequel ladite bactérie appartient au genre *Escherichia.*

4. Procédé selon la revendication 3, dans lequel ladite bactérie appartient à Escherichia coli.

5. Procédé selon la revendication 1 ou 2, dans lequel ladite bactérie appartient au genre Pantoea.

6. Procédé selon l'une quelconque des revendications 1 à 5, dans lequel ledit acide L-aminé est sélectionné dans le groupe constitué par un acide L-aminé aromatique et un acide L-aminé non aromatique.

7. Procédé selon la revendication 6, dans lequel ledit acide L-aminé est sélectionné dans le groupe constitué par la L-phénylalanine, la L-tyrosine et le L-tryptophane.

8. Procédé selon la revendication 6, dans lequel ledit acide L-aminé non aromatique est sélectionné dans le groupe constitué par la L-thréonine, la L-lysine, la L-cystéine, la L-méthionine, la L-leucine, la L-isoleucine, la L-valine, la L-histidine, la glycine, la L-sérine, la L-alanine, la L-asparagine, l'acide L-aspartique, la L-glutamine, l'acide L-glutamique, la L-proline et la L-arginine.

9. Procédé selon la revendication 8, dans lequel ledit acide L-aminé non aromatique est la L-thréonine.


### SEQUENCE LISTING

<110> Ajinomoto Co., Inc.
<120> A METHOD FOR PRODUCING AN L-AMINO ACID USING A BACTERIUM OF THE ENTEROBACTERIACEAE FAMILY WITH ENHANCED EXPRESSION OF THE fucPIKUR OPERON
<130> C486-C6134
<150> RU2005118796
   <151> 2005-06-17
<150> US60/743061
   <151> 2005-12-21
<160> 40
<170> PatentIn version 3.1
<210> 1
   <211> 1317
   <212> DNA
   <213> Escherichia coli
<220>
   <221> CDS
   <222> (1)..(1317)
<400> 1
   atg gga aac aca tca ata caa acg cag agt tac cgt gcg gta gat aaa 48
   Met Gly Asn Thr Ser Ile Gln Thr Gln Ser Tyr Arg Ala Val Asp Lys
   1 5 10 15
   gat gca ggg caa agc aga agt tac att att cca ttc gcg ctg ctg tgc 96
   Asp Ala Gly Gln Ser Arg Ser Tyr Ile Ile Pro Phe Ala Leu Leu Cys
   20 25 30
   tca ctg ttt ttt ctt tgg gcg gta gcc aat aac ctt aac gac att tta 144
   Ser Leu Phe Phe Leu Trp Ala Val Ala Asn Asn Leu Asn Asp Ile Leu
   35 40 45
   tta cct caa ttc cag cag gct ttt acg ctg aca aat ttc cag gct ggc 192
   Leu Pro Gln Phe Gln Gln Ala Phe Thr Leu Thr Asn Phe Gln Ala Gly
   50 55 60
   ctg atc caa tcg gcc ttt tac ttt ggt tat ttc att atc cca atc cct 240
   Leu Ile Gln Ser Ala Phe Tyr Phe Gly Tyr Phe Ile Ile Pro Ile Pro
   65 70 75 80
   gct ggg ata ttg atg aaa aaa ctc agt tat aaa gca ggg att att acc 288
   Ala Gly Ile Leu Met Lys Lys Leu Ser Tyr Lys Ala Gly Ile Ile Thr
   85 90 95
   ggg tta ttt tta tat gcc ttg ggt gct gca tta ttc tgg ccc gcc gca 336
   Gly Leu Phe Leu Tyr Ala Leu Gly Ala Ala Leu Phe Trp Pro Ala Ala
   100 105 110
   gaa ata atg aac tac acc ttg ttt tta gtt ggc cta ttt att att gca      384
   Glu Ile Met Asn Tyr Thr Leu Phe Leu Val Gly Leu Phe Ile Ile Ala
           115                 120                 125
   gcc gga tta ggt tgt ctg gaa act gcc gca aac cct ttt gtt acg gta      432
   Ala Gly Leu Gly Cys Leu Glu Thr Ala Ala Asn Pro Phe Val Thr Val
       130                 135                 140
   tta ggg ccg gaa agt agt ggt cac ttc cgc tta aat ctt gcg caa aca      480
   Leu Gly Pro Glu Ser Ser Gly His Phe Arg Leu Asn Leu Ala Gln Thr
   145                 150                 155                 160
   ttt aac tcg ttt ggc gca att atc gcg gtt gtc ttt ggg caa agt ctt      528
   Phe Asn Ser Phe Gly Ala Ile Ile Ala Val Val Phe Gly Gln Ser Leu
                   165                 170                 175
   att ttg tct aac gtg cca cat caa tcg caa gac gtt ctc gat aaa atg      576
   Ile Leu Ser Asn Val Pro His Gln Ser Gln Asp Val Leu Asp Lys Met
               180                 185                 190
   tct cca gag caa ttg agt gcg tat aaa cac agc ctg gta tta tcg gta      624
   Ser Pro Glu Gln Leu Ser Ala Tyr Lys His Ser Leu Val Leu Ser Val
           195                 200                 205
   cag aca cct tat atg atc atc gtg gct atc gtg tta ctg gtc gcc ctg      672
   Gln Thr Pro Tyr Met Ile Ile Val Ala Ile Val Leu Leu Val Ala Leu
       210                 215                 220
   ctg atc atg ctg acg aaa ttc ccg gca ttg cag agt gat aat cac agt      720
   Leu Ile Met Leu Thr Lys Phe Pro Ala Leu Gln Ser Asp Asn His Ser
   225                 230                 235                 240
   gac gcc aaa caa gga tcg ttc tcc gca tcg ctt tct cgc ctg gcg cgt      768
   Asp Ala Lys Gln Gly Ser Phe Ser Ala Ser Leu Ser Arg Leu Ala Arg
                   245                 250                 255
   att cgc cac tgg cgc tgg gcg gta tta gcg caa ttc tgc tat gtc ggc      816
   Ile Arg His Trp Arg Trp Ala Val Leu Ala Gln Phe Cys Tyr Val Gly
               260                 265                 270
   gca caa acg gcc tgc tgg agc tat ttg att cgc tac gct gta gaa gaa      864
   Ala Gln Thr Ala Cys Trp Ser Tyr Leu Ile Arg Tyr Ala Val Glu Glu
           275                 280                 285
   att cca ggt atg act gca ggc ttt gcc gct aac tat tta acc gga acc      912
   Ile Pro Gly Met Thr Ala Gly Phe Ala Ala Asn Tyr Leu Thr Gly Thr
       290                 295                 300
   atg gtg tgc ttc ttt att ggt cgt ttc acc ggt acc tgg ctc atc agt      960
   Met Val Cys Phe Phe Ile Gly Arg Phe Thr Gly Thr Trp Leu Ile Ser
   305                 310                 315                 320
   cgc ttc gca cca cac aaa gtc ctg gcc gcc tac gca tta atc gct atg     1008
   Arg Phe Ala Pro His Lys Val Leu Ala Ala Tyr Ala Leu Ile Ala Met
                   325                 330                 335
   gca ctg tgc ctg atc tca gcc ttc gct ggc ggt cat gtg ggc tta ata     1056
   Ala Leu Cys Leu Ile Ser Ala Phe Ala Gly Gly His Val Gly Leu Ile
               340                 345                 350
   gcc ctg act tta tgc agc gcc ttt atg tcg att cag tac cca aca atc      1104
   Ala Leu Thr Leu Cys Ser Ala Phe Met Ser Ile Gln Tyr Pro Thr Ile
           355                 360                 365
   ttc tcg ctg ggc att aag aat ctc ggc cag gac acc aaa tat ggt tcg      1152
   Phe Ser Leu Gly Ile Lys Asn Leu Gly Gln Asp Thr Lys Tyr Gly Ser
       370                 375                 380
   tcc ttc atc gtt atg acc att att ggc ggc ggt att gtc act ccg gtc      1200
   Ser Phe Ile Val Met Thr Ile Ile Gly Gly Gly Ile Val Thr Pro Val
   385                 390                 395                 400
   atg ggt ttt gtc agt gac gcg gcg ggc aac atc ccc act gct gaa ctg      1248
   Met Gly Phe Val Ser Asp Ala Ala Gly Asn Ile Pro Thr Ala Glu Leu
                   405                 410                 415
   atc ccc gca ctc tgc ttc gcg gtc atc ttt atc ttt gcc cgt ttc cgt      1296
   Ile Pro Ala Leu Cys Phe Ala Val Ile Phe Ile Phe Ala Arg Phe Arg
               420                 425                 430
   tct caa acg gca act aac tga                                          1317
   Ser Gln Thr Ala Thr Asn
           435
<210> 2
   <211> 438
   <212> PRT
   <213> Escherichia coli
<400> 2
   Met Gly Asn Thr Ser Ile Gln Thr Gln Ser Tyr Arg Ala Val Asp Lys
   1 5 10 15
   Asp Ala Gly Gln Ser Arg Ser Tyr Ile Ile Pro Phe Ala Leu Leu Cys
   20 25 30
   Ser Leu Phe Phe Leu Trp Ala Val Ala Asn Asn Leu Asn Asp Ile Leu
   35 40 45
   Leu Pro Gln Phe Gln Gln Ala Phe Thr Leu Thr Asn Phe Gln Ala Gly
   50 55 60
   Leu Ile Gln Ser Ala Phe Tyr Phe Gly Tyr Phe Ile Ile Pro Ile Pro
   65 70 75 80
   Ala Gly Ile Leu Met Lys Lys Leu Ser Tyr Lys Ala Gly Ile Ile Thr
   85 90 95
   Gly Leu Phe Leu Tyr Ala Leu Gly Ala Ala Leu Phe Trp Pro Ala Ala
   100 105 110
   Glu Ile Met Asn Tyr Thr Leu Phe Leu Val Gly Leu Phe Ile Ile Ala
   115 120 125
   Ala Gly Leu Gly Cys Leu Glu Thr Ala Ala Asn Pro Phe Val Thr Val
   130 135 140
   Leu Gly Pro Glu Ser Ser Gly His Phe Arg Leu Asn Leu Ala Gln Thr
   145 150 155 160
   Phe Asn Ser Phe Gly Ala Ile Ile Ala Val Val Phe Gly Gln Ser Leu
   165 170 175
   Ile Leu Ser Asn Val Pro His Gln Ser Gln Asp Val Leu Asp Lys Met
   180 185 190
   Ser Pro Glu Gln Leu Ser Ala Tyr Lys His Ser Leu Val Leu Ser Val
   195 200 205
   Gln Thr Pro Tyr Met Ile Ile Val Ala Ile Val Leu Leu Val Ala Leu
   210 215 220
   Leu Ile Met Leu Thr Lys Phe Pro Ala Leu Gln Ser Asp Asn His Ser
   225 230 235 240
   Asp Ala Lys Gln Gly Ser Phe Ser Ala Ser Leu Ser Arg Leu Ala Arg
   245 250 255
   Ile Arg His Trp Arg Trp Ala Val Leu Ala Gln Phe Cys Tyr Val Gly
   260 265 270
   Ala Gln Thr Ala Cys Trp Ser Tyr Leu Ile Arg Tyr Ala Val Glu Glu
   275 280 285
   Ile Pro Gly Met Thr Ala Gly Phe Ala Ala Asn Tyr Leu Thr Gly Thr
   290 295 300
   Met Val Cys Phe Phe Ile Gly Arg Phe Thr Gly Thr Trp Leu Ile Ser
   305 310 315 320
   Arg Phe Ala Pro His Lys Val Leu Ala Ala Tyr Ala Leu Ile Ala Met
   325 330 335
   Ala Leu Cys Leu Ile Ser Ala Phe Ala Gly Gly His Val Gly Leu Ile
   340 345 350
   Ala Leu Thr Leu Cys Ser Ala Phe Met Ser Ile Gln Tyr Pro Thr Ile
   355 360 365
   Phe Ser Leu Gly Ile Lys Asn Leu Gly Gln Asp Thr Lys Tyr Gly Ser
   370 375 380
   Ser Phe Ile Val Met Thr Ile Ile Gly Gly Gly Ile Val Thr Pro Val
   385 390 395 400
   Met Gly Phe Val Ser Asp Ala Ala Gly Asn Ile Pro Thr Ala Glu Leu
   405 410 415
   Ile Pro Ala Leu Cys Phe Ala Val Ile Phe Ile Phe Ala Arg Phe Arg
   420 425 430
   Ser Gln Thr Ala Thr Asn
   435
<210> 3
   <211> 1776
   <212> DNA
   <213> Escherichia coli
<220>
   <221> CDS
   <222> (1)..(1776)
<400> 3
   atg aaa aaa atc agc tta ccg aaa att ggt atc cgc ccg gtt att gac        48
   Met Lys Lys Ile Ser Leu Pro Lys Ile Gly Ile Arg Pro Val Ile Asp
   1               5                   10                  15
   ggt cgt cgc atg ggt gtt cgt gag tcg ctt gaa gaa caa aca atg aat        96
   Gly Arg Arg Met Gly Val Arg Glu Ser Leu Glu Glu Gln Thr Met Asn
               20                  25                  30
   atg gcg aaa gct acg gcc gca ctg ctg acc gag aaa ctg cgc cat gcc        144
   Met Ala Lys Ala Thr Ala Ala Leu Leu Thr Glu Lys Leu Arg His Ala
           35                  40                  45
   tgc gga gct gcc gtc gag tgt gtc att tcc gat acc tgt atc gcg ggt        192
   Cys Gly Ala Ala Val Glu Cys Val Ile Ser Asp Thr Cys Ile Ala Gly
       50                  55                  60
   atg gct gaa gcc gct gct tgc gaa gaa aaa ttc agc agt cag aat gta        240
   Met Ala Glu Ala Ala Ala Cys Glu Glu Lys Phe Ser Ser Gln Asn Val
   65                  70                  75                  80
   ggc ctc acc att acg gta acg cct tgc tgg tgc tat ggc agt gaa acc        288
   Gly Leu Thr Ile Thr Val Thr Pro Cys Trp Cys Tyr Gly Ser Glu Thr
                   85                  90                  95
   atc gac atg gat cca acc cgc ccg aag gcc att tgg ggc ttt aac ggc        336
   Ile Asp Met Asp Pro Thr Arg Pro Lys Ala Ile Trp Gly Phe Asn Gly
               100                 105                 110
   act gaa cgc ccc ggc gct gtt tac ctg gca gcg gct ctg gca gct cac        384
   Thr Glu Arg Pro Gly Ala Val Tyr Leu Ala Ala Ala Leu Ala Ala His
           115                 120                 125
   agc cag aaa ggc atc cca gca ttc tcc att tac ggt cat gac gtt cag        432
   Ser Gln Lys Gly Ile Pro Ala Phe Ser Ile Tyr Gly His Asp Val Gln
       130                 135                 140
   gat gcc gat gac aca tcg att cct gcc gat gtt gaa gaa aaa ctg ctg        480
   Asp Ala Asp Asp Thr Ser Ile Pro Ala Asp Val Glu Glu Lys Leu Leu
   145                 150                 155                 160
   cgc ttt gcc cgc gcc ggt ttg gcc gtc gcc agc atg aaa ggt aaa agc        528
   Arg Phe Ala Arg Ala Gly Leu Ala Val Ala Ser Met Lys Gly Lys Ser
                   165                 170                 175
   tat ctg tcg ctg ggc ggc gtt tcg atg ggt atc gcc ggt tcc att gtt        576
   Tyr Leu Ser Leu Gly Gly Val Ser Met Gly Ile Ala Gly Ser Ile Val
               180                 185                 190
   gat cac aac ttc ttt gaa tcc tgg ctg gga atg aaa gtc cag gcg gtg        624
   Asp His Asn Phe Phe Glu Ser Trp Leu Gly Met Lys Val Gln Ala Val
           195                 200                 205
   gat atg acc gaa ctg cgt cgc cgt atc gat cag aag att tac gac gaa      672
   Asp Met Thr Glu Leu Arg Arg Arg Ile Asp Gln Lys Ile Tyr Asp Glu
       210                 215                 220
   gcc gaa ttg gaa atg gca ctg gcc tgg gct gat aaa aac ttc cgc tat      720
   Ala Glu Leu Glu Met Ala Leu Ala Trp Ala Asp Lys Asn Phe Arg Tyr
   225                 230                 235                 240
   ggc gaa gat gaa aat aac aaa cag tat caa cgt aat gcc gag caa agc      768
   Gly Glu Asp Glu Asn Asn Lys Gln Tyr Gln Arg Asn Ala Glu Gln Ser
                   245                 250                 255
   cgc gca gtt ctg cgc gaa agt tta ctg atg gcg atg tgt atc cgc gac      816
   Arg Ala Val Leu Arg Glu Ser Leu Leu Met Ala Met Cys Ile Arg Asp
               260                 265                 270
   atg atg caa ggc aac agc aaa ctg gcc gat att ggt cgc gtg gaa gaa      864
   Met Met Gln Gly Asn Ser Lys Leu Ala Asp Ile Gly Arg Val Glu Glu
           275                 280                 285
   tca ctt ggc tac aac gcc atc gct gcg ggc ttc cag ggg caa cgt cac      912
   Ser Leu Gly Tyr Asn Ala Ile Ala Ala Gly Phe Gln Gly Gln Arg His
       290                 295                 300
   tgg acc gat caa tat ccc aat ggt gac acc gcc gaa gcg atc ctc aac      960
   Trp Thr Asp Gln Tyr Pro Asn Gly Asp Thr Ala Glu Ala Ile Leu Asn
   305                 310                 315                 320
   agt tca ttt gac tgg aat ggc gtg cgc gaa ccc ttt gtc gtg gcg acc     1008
   Ser Ser Phe Asp Trp Asn Gly Val Arg Glu Pro Phe Val Val Ala Thr
                   325                 330                 335
   gaa aac gac agt ctt aac ggc gtg gca atg cta atg ggt cac cag ctc     1056
   Glu Asn Asp Ser Leu Asn Gly Val Ala Met Leu Met Gly His Gln Leu
               340                 345                 350
   acc ggc acc gct cag gta ttt gcc gat gtg cgt acc tac tgg tca cca     1104
   Thr Gly Thr Ala Gln Val Phe Ala Asp Val Arg Thr Tyr Trp Ser Pro
           355                 360                 365
   gaa gca att gag cgt gta acg ggg cat aaa ctg gat gga ctg gca gaa     1152
   Glu Ala Ile Glu Arg Val Thr Gly His Lys Leu Asp Gly Leu Ala Glu
       370                 375                 380
   cac ggc atc atc cat ttg atc aac tcc ggt tct gct gcg ctg gac ggt     1200
   His Gly Ile Ile His Leu Ile Asn Ser Gly Ser Ala Ala Leu Asp Gly
   385                 390                 395                 400
   tcc tgt aaa caa cgc gac agc gaa ggt aac ccg acg atg aag cca cac     1248
   Ser Cys Lys Gln Arg Asp Ser Glu Gly Asn Pro Thr Met Lys Pro His
                   405                 410                 415
   tgg gaa atc tct cag caa gag gct gac gct tgc ctc gcc gct acc gaa     1296
   Trp Glu Ile Ser Gln Gln Glu Ala Asp Ala Cys Leu Ala Ala Thr Glu
               420                 425                 430
   tgg tgc ccg gcg atc cac gaa tac ttc cgt ggc ggc ggt tac tct tcc      1344
   Trp Cys Pro Ala Ile His Glu Tyr Phe Arg Gly Gly Gly Tyr Ser Ser
           435                 440                 445
   cgc ttc ctt acc gaa ggc ggc gtc ccg ttc acc atg act cgt gtc aac      1392
   Arg Phe Leu Thr Glu Gly Gly Val Pro Phe Thr Met Thr Arg Val Asn
       450                 455                 460
   atc atc aaa ggc ctg gga ccg gta ctg caa atc gcg gaa ggc tgg agc      1440
   Ile Ile Lys Gly Leu Gly Pro Val Leu Gln Ile Ala Glu Gly Trp Ser
   465                 470                 475                 480
   gtg gaa ttg ccg aag gat gtg cat gac atc ctc aac aaa cgc acc aac      1488
   Val Glu Leu Pro Lys Asp Val His Asp Ile Leu Asn Lys Arg Thr Asn
                   485                 490                 495
   tca acc tgg cca acc acc tgg ttt gca ccg cgc ctc acc ggt aaa ggg      1536
   Ser Thr Trp Pro Thr Thr Trp Phe Ala Pro Arg Leu Thr Gly Lys Gly
               500                 505                 510
   ccg ttt acg gat gtg tac tcg gta atg gcg aac tgg ggc gct aac cat      1584
   Pro Phe Thr Asp Val Tyr Ser Val Met Ala Asn Trp Gly Ala Asn His
           515                 520                 525
   ggg gtt ctg acc atc ggc cac gtt ggc gca gac ttt atc act ctc gcc      1632
   Gly Val Leu Thr Ile Gly His Val Gly Ala Asp Phe Ile Thr Leu Ala
       530                 535                 540
   tcc atg ctg cgt atc ccg gta tgt atg cac aac gtt gaa gag acc aaa      1680
   Ser Met Leu Arg Ile Pro Val Cys Met His Asn Val Glu Glu Thr Lys
   545                 550                 555                 560
   gtg tat cgt cct tct gcc tgg gct gcg cac ggc atg gat att gaa ggc      1728
   Val Tyr Arg Pro Ser Ala Trp Ala Ala His Gly Met Asp Ile Glu Gly
                   565                 570                 575
   cag gat tac cgc gct tgc cag aac tac ggt ccg ttg tac aag cgt taa      1776
   Gln Asp Tyr Arg Ala Cys Gln Asn Tyr Gly Pro Leu Tyr Lys Arg
               580                 585                 590
<210> 4
   <211> 591
   <212> PRT
   <213> Escherichia coli
<400> 4
   Met Lys Lys Ile Ser Leu Pro Lys Ile Gly Ile Arg Pro Val Ile Asp
   1               5                   10                  15
   Gly Arg Arg Met Gly Val Arg Glu Ser Leu Glu Glu Gln Thr Met Asn
               20                  25                  30
   Met Ala Lys Ala Thr Ala Ala Leu Leu Thr Glu Lys Leu Arg His Ala
           35                  40                  45
   Cys Gly Ala Ala Val Glu Cys Val Ile Ser Asp Thr Cys Ile Ala Gly
       50                  55                  60
   Met Ala Glu Ala Ala Ala Cys Glu Glu Lys Phe Ser Ser Gln Asn Val
   65 70 75 80
   Gly Leu Thr Ile Thr Val Thr Pro Cys Trp Cys Tyr Gly Ser Glu Thr
   85 90 95
   Ile Asp Met Asp Pro Thr Arg Pro Lys Ala Ile Trp Gly Phe Asn Gly
   100 105 110
   Thr Glu Arg Pro Gly Ala Val Tyr Leu Ala Ala Ala Leu Ala Ala His
   115 120 125
   Ser Gln Lys Gly Ile Pro Ala Phe Ser Ile Tyr Gly His Asp Val Gln
   130 135 140
   Asp Ala Asp Asp Thr Ser Ile Pro Ala Asp Val Glu Glu Lys Leu Leu
   145 150 155 160
   Arg Phe Ala Arg Ala Gly Leu Ala Val Ala Ser Met Lys Gly Lys Ser
   165 170 175
   Tyr Leu Ser Leu Gly Gly Val Ser Met Gly Ile Ala Gly Ser Ile Val
   180 185 190
   Asp His Asn Phe Phe Glu Ser Trp Leu Gly Met Lys Val Gln Ala Val
   195 200 205
   Asp Met Thr Glu Leu Arg Arg Arg Ile Asp Gln Lys Ile Tyr Asp Glu
   210 215 220
   Ala Glu Leu Glu Met Ala Leu Ala Trp Ala Asp Lys Asn Phe Arg Tyr
   225 230 235 240
   Gly Glu Asp Glu Asn Asn Lys Gln Tyr Gln Arg Asn Ala Glu Gln Ser
   245 250 255
   Arg Ala Val Leu Arg Glu Ser Leu Leu Met Ala Met Cys Ile Arg Asp
   260 265 270
   Met Met Gln Gly Asn Ser Lys Leu Ala Asp Ile Gly Arg Val Glu Glu
   275 280 285
   Ser Leu Gly Tyr Asn Ala Ile Ala Ala Gly Phe Gln Gly Gln Arg His
   290 295 300
   Trp Thr Asp Gln Tyr Pro Asn Gly Asp Thr Ala Glu Ala Ile Leu Asn
   305 310 315 320
   Ser Ser Phe Asp Trp Asn Gly Val Arg Glu Pro Phe Val Val Ala Thr
   325 330 335
   Glu Asn Asp Ser Leu Asn Gly Val Ala Met Leu Met Gly His Gln Leu
   340 345 350
   Thr Gly Thr Ala Gln Val Phe Ala Asp Val Arg Thr Tyr Trp Ser Pro
   355 360 365
   Glu Ala Ile Glu Arg Val Thr Gly His Lys Leu Asp Gly Leu Ala Glu
   370 375 380
   His Gly Ile Ile His Leu Ile Asn Ser Gly Ser Ala Ala Leu Asp Gly
   385 390 395 400
   Ser Cys Lys Gln Arg Asp Ser Glu Gly Asn Pro Thr Met Lys Pro His
   405 410 415
   Trp Glu Ile Ser Gln Gln Glu Ala Asp Ala Cys Leu Ala Ala Thr Glu
   420 425 430
   Trp Cys Pro Ala Ile His Glu Tyr Phe Arg Gly Gly Gly Tyr Ser Ser
   435 440 445
   Arg Phe Leu Thr Glu Gly Gly Val Pro Phe Thr Met Thr Arg Val Asn
   450 455 460
   Ile Ile Lys Gly Leu Gly Pro Val Leu Gln Ile Ala Glu Gly Trp Ser
   465 470 475 480
   Val Glu Leu Pro Lys Asp Val His Asp Ile Leu Asn Lys Arg Thr Asn
   485 490 495
   Ser Thr Trp Pro Thr Thr Trp Phe Ala Pro Arg Leu Thr Gly Lys Gly
   500 505 510
   Pro Phe Thr Asp Val Tyr Ser Val Met Ala Asn Trp Gly Ala Asn His
   515 520 525
   Gly Val Leu Thr Ile Gly His Val Gly Ala Asp Phe Ile Thr Leu Ala
   530 535 540
   Ser Met Leu Arg Ile Pro Val Cys Met His Asn Val Glu Glu Thr Lys
   545 550 555 560
   Val Tyr Arg Pro Ser Ala Trp Ala Ala His Gly Met Asp Ile Glu Gly
   565 570 575
   Gln Asp Tyr Arg Ala Cys Gln Asn Tyr Gly Pro Leu Tyr Lys Arg
   580 585 590
<210> 5
   <211> 1449
   <212> DNA
   <213> Escherichia coli
<220>
<221> CDS
   <222> (1)..(1449)
<400> 5
   atg tta tcc ggc tat att gca gga gcg att atg aaa caa gaa gtt atc      48
   Met Leu Ser Gly Tyr Ile Ala Gly Ala Ile Met Lys Gln Glu Val Ile
   1               5                   10                  15
   ctg gta ctc gac tgt ggc gcg acc aat gtc agg gcc atc gcg gtt aat      96
   Leu Val Leu Asp Cys Gly Ala Thr Asn Val Arg Ala Ile Ala Val Asn
               20                  25                  30
   cgg cag ggc aaa att gtt gcc cgc gcc tca acg cct aat gcc agc gat      144
   Arg Gln Gly Lys Ile Val Ala Arg Ala Ser Thr Pro Asn Ala Ser Asp
           35                  40                  45
   atc gcg atg gaa aac aac acc tgg cac cag tgg tct tta gac gcc att      192
   Ile Ala Met Glu Asn Asn Thr Trp His Gln Trp Ser Leu Asp Ala Ile
       50                  55                  60
   ttg caa cgc ttt gct gat tgc tgt cgg caa atc aat agt gaa ctg act      240
   Leu Gln Arg Phe Ala Asp Cys Cys Arg Gln Ile Asn Ser Glu Leu Thr
   65                  70                  75                  80
   gaa tgc cac atc cgc ggt atc gcc gtc acc acc ttt ggt gtg gat ggc      288
   Glu Cys His Ile Arg Gly Ile Ala Val Thr Thr Phe Gly Val Asp Gly
                   85                  90                  95
   gct ctg gta gat aag caa ggc aat ctg ctc tat ccg att att agc tgg      336
   Ala Leu Val Asp Lys Gln Gly Asn Leu Leu Tyr Pro Ile Ile Ser Trp
               100                 105                 110
   aaa tgt ccg cga aca gca gcg gtt atg gac aat att gaa cgg tta atc      384
   Lys Cys Pro Arg Thr Ala Ala Val Met Asp Asn Ile Glu Arg Leu Ile
           115                 120                 125
   tcc gca cag cgg ttg cag gct att tct ggc gtc gga gcc ttt agt ttc      432
   Ser Ala Gln Arg Leu Gln Ala Ile Ser Gly Val Gly Ala Phe Ser Phe
       130                 135                 140
   aat acg tta tat aag ttg gtg tgg ttg aaa gaa aat cat cca caa ctg      480
   Asn Thr Leu Tyr Lys Leu Val Trp Leu Lys Glu Asn His Pro Gln Leu
   145                 150                 155                 160
   ctg gaa cgc gcg cac gcc tgg ctc ttt att tcg tcg ctg att aac cac      528
   Leu Glu Arg Ala His Ala Trp Leu Phe Ile Ser Ser Leu Ile Asn His
                   165                 170                 175
   cgt tta acc ggc gaa ttc act act gat atc acg atg gcc gga acc agc      576
   Arg Leu Thr Gly Glu Phe Thr Thr Asp Ile Thr Met Ala Gly Thr Ser
               180                 185                 190
   cag atg ctg gat atc cag caa cgc gat ttc agt ccg caa att tta caa      624
   Gln Met Leu Asp Ile Gln Gln Arg Asp Phe Ser Pro Gln Ile Leu Gln
           195                 200                 205
   gcc acc ggt att cca cgc cga ctc ttc cct cgt ctg gtg gaa gcg ggt      672
   Ala Thr Gly Ile Pro Arg Arg Leu Phe Pro Arg Leu Val Glu Ala Gly
       210                 215                 220
   gaa cag att ggt acg cta cag aac agc gcc gca gca atg ctc ggc tta      720
   Glu Gln Ile Gly Thr Leu Gln Asn Ser Ala Ala Ala Met Leu Gly Leu
   225                 230                 235                 240
   ccc gtt ggc ata ccg gtg att tcc gca ggt cac gat acc cag ttc gcc      768
   Pro Val Gly Ile Pro Val Ile Ser Ala Gly His Asp Thr Gln Phe Ala
                   245                 250                 255
   ctt ttt ggc gct ggt gct gaa caa aat gaa ccc gtg ctc tct tcc ggt      816
   Leu Phe Gly Ala Gly Ala Glu Gln Asn Glu Pro Val Leu Ser Ser Gly
               260                 265                 270
   aca tgg gaa att tta atg gtt cgc agc gcc cag gtt gat act tcg ctg      864
   Thr Trp Glu Ile Leu Met Val Arg Ser Ala Gln Val Asp Thr Ser Leu
           275                 280                 285
   tta agt cag tac gcc ggt tcc acc tgc gaa ctg gat agc cag gca ggg      912
   Leu Ser Gln Tyr Ala Gly Ser Thr Cys Glu Leu Asp Ser Gln Ala Gly
       290                 295                 300
   ttg tat aac cca ggt atg caa tgg ctg gca tcc ggc gtg ctg gaa tgg      960
   Leu Tyr Asn Pro Gly Met Gln Trp Leu Ala Ser Gly Val Leu Glu Trp
   305                 310                 315                 320
   gtg aga aaa ctg ttc tgg acg gct gaa aca ccc tgg caa atg ttg att     1008
   Val Arg Lys Leu Phe Trp Thr Ala Glu Thr Pro Trp Gln Met Leu Ile
                   325                 330                 335
   gaa gaa gct cgt ctg atc gcg cct ggc gcg gat ggc gta aaa atg cag     1056
   Glu Glu Ala Arg Leu Ile Ala Pro Gly Ala Asp Gly Val Lys Met Gln
               340                 345                 350
   tgt gat tta ttg tcg tgt cag aac gct ggc tgg caa gga gtg acg ctt     1104
   Cys Asp Leu Leu Ser Cys Gln Asn Ala Gly Trp Gln Gly Val Thr Leu
           355                 360                 365
   aat acc acg cgg ggg cat ttc tat cgc gcg gcg ctg gaa ggg tta act     1152
   Asn Thr Thr Arg Gly His Phe Tyr Arg Ala Ala Leu Glu Gly Leu Thr
       370                 375                 380
   gcg caa tta cag cgc aat cta cag atg ctg gaa aaa atc ggg cac ttt     1200
   Ala Gln Leu Gln Arg Asn Leu Gln Met Leu Glu Lys Ile Gly His Phe
   385                 390                 395                 400
   aag gcc tct gaa tta ttg tta gtc ggt gga gga agt cgc aac aca ttg     1248
   Lys Ala Ser Glu Leu Leu Leu Val Gly Gly Gly Ser Arg Asn Thr Leu
                   405                 410                 415
   tgg aat cag att aaa gcc aat atg ctt gat att ccg gta aaa gtt ctc     1296
   Trp Asn Gln Ile Lys Ala Asn Met Leu Asp Ile Pro Val Lys Val Leu
               420                 425                 430
   gac gac gcc gaa acg acc gtc gca gga gct gcg ctg ttc ggt tgg tat     1344
   Asp Asp Ala Glu Thr Thr Val Ala Gly Ala Ala Leu Phe Gly Trp Tyr
           435                 440                 445
   ggc gta ggg gaa ttt aac agc ccg gaa gaa gcc cgc gca cag att cat     1392
   Gly Val Gly Glu Phe Asn Ser Pro Glu Glu Ala Arg Ala Gln Ile His
       450                 455                 460
   tat cag tac cgt tat ttc tac ccg caa act gaa cct gaa ttt ata gag     1440
   Tyr Gln Tyr Arg Tyr Phe Tyr Pro Gln Thr Glu Pro Glu Phe Ile Glu
   465                 470                 475                 480
   gaa gtg tga                                                         1449
   Glu Val
<210> 6
   <211> 482
   <212> PRT
   <213> Escherichia coli
<400> 6
   Met Leu Ser Gly Tyr Ile Ala Gly Ala Ile Met Lys Gln Glu Val Ile
   1               5                   10                  15
   Leu Val Leu Asp Cys Gly Ala Thr Asn Val Arg Ala Ile Ala Val Asn
               20                  25                  30
   Arg Gln Gly Lys Ile Val Ala Arg Ala Ser Thr Pro Asn Ala Ser Asp
           35                  40                  45
   Ile Ala Met Glu Asn Asn Thr Trp His Gln Trp Ser Leu Asp Ala Ile
       50                  55                  60
   Leu Gln Arg Phe Ala Asp Cys Cys Arg Gln Ile Asn Ser Glu Leu Thr
   65                  70                  75                  80
   Glu Cys His Ile Arg Gly Ile Ala Val Thr Thr Phe Gly Val Asp Gly
                   85                  90                  95
   Ala Leu Val Asp Lys Gln Gly Asn Leu Leu Tyr Pro Ile Ile Ser Trp
               100                 105                 110
   Lys Cys Pro Arg Thr Ala Ala Val Met Asp Asn Ile Glu Arg Leu Ile
           115                 120                 125
   Ser Ala Gln Arg Leu Gln Ala Ile Ser Gly Val Gly Ala Phe Ser Phe
       130                 135                 140
   Asn Thr Leu Tyr Lys Leu Val Trp Leu Lys Glu Asn His Pro Gln Leu
   145                 150                 155                 160
   Leu Glu Arg Ala His Ala Trp Leu Phe Ile Ser Ser Leu Ile Asn His
                   165                 170                 175
   Arg Leu Thr Gly Glu Phe Thr Thr Asp Ile Thr Met Ala Gly Thr Ser
               180                 185                 190
   Gln Met Leu Asp Ile Gln Gln Arg Asp Phe Ser Pro Gln Ile Leu Gln
           195                 200                 205
   Ala Thr Gly Ile Pro Arg Arg Leu Phe Pro Arg Leu Val Glu Ala Gly
       210                 215                 220
   Glu Gln Ile Gly Thr Leu Gln Asn Ser Ala Ala Ala Met Leu Gly Leu
   225                 230                 235                 240
   Pro Val Gly Ile Pro Val Ile Ser Ala Gly His Asp Thr Gln Phe Ala
                   245                 250                 255
   Leu Phe Gly Ala Gly Ala Glu Gln Asn Glu Pro Val Leu Ser Ser Gly
               260                 265                 270
   Thr Trp Glu Ile Leu Met Val Arg Ser Ala Gln Val Asp Thr Ser Leu
           275                 280                 285
   Leu Ser Gln Tyr Ala Gly Ser Thr Cys Glu Leu Asp Ser Gln Ala Gly
   290 295 300
   Leu Tyr Asn Pro Gly Met Gln Trp Leu Ala Ser Gly Val Leu Glu Trp
   305 310 315 320
   Val Arg Lys Leu Phe Trp Thr Ala Glu Thr Pro Trp Gln Met Leu Ile
   325 330 335
   Glu Glu Ala Arg Leu Ile Ala Pro Gly Ala Asp Gly Val Lys Met Gln
   340 345 350
   Cys Asp Leu Leu Ser Cys Gln Asn Ala Gly Trp Gln Gly Val Thr Leu
   355 360 365
   Asn Thr Thr Arg Gly His Phe Tyr Arg Ala Ala Leu Glu Gly Leu Thr
   370 375 380
   Ala Gln Leu Gln Arg Asn Leu Gln Met Leu Glu Lys Ile Gly His Phe
   385 390 395 400
   Lys Ala Ser Glu Leu Leu Leu Val Gly Gly Gly Ser Arg Asn Thr Leu
   405 410 415
   Trp Asn Gln Ile Lys Ala Asn Met Leu Asp Ile Pro Val Lys Val Leu
   420 425 430
   Asp Asp Ala Glu Thr Thr Val Ala Gly Ala Ala Leu Phe Gly Trp Tyr
   435 440 445
   Gly Val Gly Glu Phe Asn Ser Pro Glu Glu Ala Arg Ala Gln Ile His
   450 455 460
   Tyr Gln Tyr Arg Tyr Phe Tyr Pro Gln Thr Glu Pro Glu Phe Ile Glu
   465 470 475 480
   Glu Val
<210> 7
   <211> 423
   <212> DNA
   <213> Escherichia coli
<220>
   <221> CDS
   <222> (1)..(423)
<400> 7
   atg ctg aaa aca att tcg ccg tta att tct ccc gaa cta ttg aaa gtg      48
   Met Leu Lys Thr Ile Ser Pro Leu Ile Ser Pro Glu Leu Leu Lys Val
   1               5                   10                  15
   ctg gca gag atg gga cat gga gat gaa att att ttt tcc gat gct cac      96
   Leu Ala Glu Met Gly His Gly Asp Glu Ile Ile Phe Ser Asp Ala His
               20                  25                  30
   ttt ccc gcc cat tcg atg gga ccg cag gtg atc cgc gct gat ggc ctg      144
   Phe Pro Ala His Ser Met Gly Pro Gln Val Ile Arg Ala Asp Gly Leu
           35                  40                  45
   ttg gtg agc gac ttg ctc cag gcg att atc ccg tta ttt gaa ctg gac      192
   Leu Val Ser Asp Leu Leu Gln Ala Ile Ile Pro Leu Phe Glu Leu Asp
       50                  55                  60
   agt tat gca ccg ccg ctg gtg atg atg gcg gcg gta gaa ggt gac act      240
   Ser Tyr Ala Pro Pro Leu Val Met Met Ala Ala Val Glu Gly Asp Thr
   65                  70                  75                  80
   ctc gat cct gaa gta gaa cga cgt tac cgt aat gcg ctt tca cta caa      288
   Leu Asp Pro Glu Val Glu Arg Arg Tyr Arg Asn Ala Leu Ser Leu Gln
                   85                  90                  95
   gcc ccg tgt cct gac atc atc cgc atc aat cgt ttt gcg ttt tat gaa      336
   Ala Pro Cys Pro Asp Ile Ile Arg Ile Asn Arg Phe Ala Phe Tyr Glu
               100                 105                 110
   cgg gcg caa aaa gcc ttt gcg atc gtt atc aca ggc gaa cga gcg aag      384
   Arg Ala Gln Lys Ala Phe Ala Ile Val Ile Thr Gly Glu Arg Ala Lys
           115                 120                 125
   tac ggg aat att ctt tta aaa aaa ggg gta aca ccg taa                  423
   Tyr Gly Asn Ile Leu Leu Lys Lys Gly Val Thr Pro
       130                 135                 140
<210> 8
   <211> 140
   <212> PRT
   <213> Escherichia coli
<400> 8
   Met Leu Lys Thr Ile Ser Pro Leu Ile Ser Pro Glu Leu Leu Lys Val
   1               5                   10                  15
   Leu Ala Glu Met Gly His Gly Asp Glu Ile Ile Phe Ser Asp Ala His
               20                  25                  30
   Phe Pro Ala His Ser Met Gly Pro Gln Val Ile Arg Ala Asp Gly Leu
           35                  40                  45
   Leu Val Ser Asp Leu Leu Gln Ala Ile Ile Pro Leu Phe Glu Leu Asp
       50                  55                  60
   Ser Tyr Ala Pro Pro Leu Val Met Met Ala Ala Val Glu Gly Asp Thr
   65                  70                  75                  80
   Leu Asp Pro Glu Val Glu Arg Arg Tyr Arg Asn Ala Leu Ser Leu Gln
                   85                  90                  95
   Ala Pro Cys Pro Asp Ile Ile Arg Ile Asn Arg Phe Ala Phe Tyr Glu
               100                 105                 110
   Arg Ala Gln Lys Ala Phe Ala Ile Val Ile Thr Gly Glu Arg Ala Lys
           115                 120                 125
   Tyr Gly Asn Ile Leu Leu Lys Lys Gly Val Thr Pro
       130                 135                 140
<210> 9
   <211> 732
   <212> DNA
   <213> Escherichia coil
<220>
   <221> CDS
   <222> (1)..(732)
<400> 9
   atg aaa gcg gca cgc cag caa gcg ata gtc gac ctg ctg ctg aac cat      48
   Met Lys Ala Ala Arg Gln Gln Ala Ile Val Asp Leu Leu Leu Asn His
   1               5                   10                  15
   acc agc ctg acc acg gaa gct ctc tct gaa cag cta aag gtc agt aaa      96
   Thr Ser Leu Thr Thr Glu Ala Leu Ser Glu Gln Leu Lys Val Ser Lys
               20                  25                  30
   gaa acc att cgt cgc gat ctc aat gaa tta cag acg cag ggt aaa att     144
   Glu Thr Ile Arg Arg Asp Leu Asn Glu Leu Gln Thr Gln Gly Lys Ile
           35                  40                  45
   ctg cgc aat cat gga cgc gct aaa tat atc cac cgt caa aat caa gac     192
   Leu Arg Asn His Gly Arg Ala Lys Tyr Ile His Arg Gln Asn Gln Asp
       50                  55                  60
   agt ggc gat ccc ttt cac atc agg ctg aaa agc cat tat gcg cat aaa     240
   Ser Gly Asp Pro Phe His Ile Arg Leu Lys Ser His Tyr Ala His Lys
   65                  70                  75                  80
   gca gat atc gcg cgc gag gcg ctc gcg tgg att gaa gaa ggg atg gtg     288
   Ala Asp Ile Ala Arg Glu Ala Leu Ala Trp Ile Glu Glu Gly Met Val
                   85                  90                  95
   ata gcc tta gac gcc agt tca act tgc tgg tat ctg gca cgc cag ttg     336
   Ile Ala Leu Asp Ala Ser Ser Thr Cys Trp Tyr Leu Ala Arg Gln Leu
               100                 105                 110
   cct gac atc aac att cag gtc ttc acc aat agc cat ccg att tgc cat     384
   Pro Asp Ile Asn Ile Gln Val Phe Thr Asn Ser His Pro Ile Cys His
           115                 120                 125
   gaa ctc ggt aaa cgc gaa cgc att caa ctg atc agt tcc ggc ggc aca     432
   Glu Leu Gly Lys Arg Glu Arg Ile Gln Leu Ile Ser Ser Gly Gly Thr
       130                 135                 140
   ctt gag cgc aaa tat ggc tgt tac gtc aat ccc tcg ctg att tcc caa     480
   Leu Glu Arg Lys Tyr Gly Cys Tyr Val Asn Pro Ser Leu Ile Ser Gln
   145                 150                 155                 160
   ctt aaa tcg ctg gaa atc gat ctg ttt att ttt tct tgt gaa ggg atc     528
   Leu Lys Ser Leu Glu Ile Asp Leu Phe Ile Phe Ser Cys Glu Gly Ile
                   165                 170                 175
   gat agc agc ggc gca ctg tgg gac tcc aat gcg atc aac gct gat tac     576
   Asp Ser Ser Gly Ala Leu Trp Asp Ser Asn Ala Ile Asn Ala Asp Tyr
               180                 185                 190
   aaa tcg atg cta tta aaa cgt gcc gcg caa tcg ttg tta ttg att gat      624
   Lys Ser Met Leu Leu Lys Arg Ala Ala Gln Ser Leu Leu Leu Ile Asp
           195                 200                 205
   aaa agt aaa ttt aat cgt tca ggg gaa gcc cgc atc ggg cat ctg gat      672
   Lys Ser Lys Phe Asn Arg Ser Gly Glu Ala Arg Ile Gly His Leu Asp
       210                 215                 220
   gag gta acg cac att att tct gat gag cgc cag gtt gca act tct ttg      720
   Glu Val Thr His Ile Ile Ser Asp Glu Arg Gln Val Ala Thr Ser Leu
   225                 230                 235                 240
   gta aca gcc tga                                                      732
   Val Thr Ala
<210> 10
   <211> 243
   <212> PRT
   <213> Escherichia coli
<400> 10
   Met Lys Ala Ala Arg Gln Gln Ala Ile Val Asp Leu Leu Leu Asn His
   1               5                   10                  15
   Thr Ser Leu Thr Thr Glu Ala Leu Ser Glu Gln Leu Lys Val Ser Lys
               20                  25                  30
   Glu Thr Ile Arg Arg Asp Leu Asn Glu Leu Gln Thr Gln Gly Lys Ile
           35                  40                  45
   Leu Arg Asn His Gly Arg Ala Lys Tyr Ile His Arg Gln Asn Gln Asp
       50                  55                  60
   Ser Gly Asp Pro Phe His Ile Arg Leu Lys Ser His Tyr Ala His Lys
   65                  70                  75                  80
   Ala Asp Ile Ala Arg Glu Ala Leu Ala Trp Ile Glu Glu Gly Met Val
                   85                  90                  95
   Ile Ala Leu Asp Ala Ser Ser Thr Cys Trp Tyr Leu Ala Arg Gln Leu
               100                 105                 110
   Pro Asp Ile Asn Ile Gln Val Phe Thr Asn Ser His Pro Ile Cys His
           115                 120                 125
   Glu Leu Gly Lys Arg Glu Arg Ile Gln Leu Ile Ser Ser Gly Gly Thr
       130                 135                 140
   Leu Glu Arg Lys Tyr Gly Cys Tyr Val Asn Pro Ser Leu Ile Ser Gln
   145                 150                 155                 160
   Leu Lys Ser Leu Glu Ile Asp Leu Phe Ile Phe Ser Cys Glu Gly Ile
                   165                 170                 175
   Asp Ser Ser Gly Ala Leu Trp Asp Ser Asn Ala Ile Asn Ala Asp Tyr
               180                 185                 190
   Lys Ser Met Leu Leu Lys Arg Ala Ala Gln Ser Leu Leu Leu Ile Asp
           195                 200                 205
   Lys Ser Lys Phe Asn Arg Ser Gly Glu Ala Arg Ile Gly His Leu Asp
       210                 215                 220
   Glu Val Thr His Ile Ile Ser Asp Glu Arg Gln Val Ala Thr Ser Leu
   225                 230                 235                 240
   Val Thr Ala
<210> 11
   <211> 120
   <212> DNA
   <213> Artificial
<220>
   <223> DNA fragment containing attL
<400> 11
   agatcttgaa gcctgctttt ttatactaag ttggcattat aaaaaagcat tgcttatcaa 60
   tttgttgcaa cgaacaggtc actatcagtc aaaataaaat cattatttga tttcgaattc 120
<210> 12
   <211> 40
   <212> DNA
   <213> Artificial
<220>
   <223> primer
<400> 12
   ctagtaagat cttgaagcct gcttttttat actaagttgg 40
<210> 13
   <211> 41
   <212> DNA
   <213> Artificial
<220>
   <223> primer
<400> 13
   atgatcgaat tcgaaatcaa ataatgattt tattttgact g 41
<210> 14
   <211> 184
   <212> DNA
   <213> Artificial
<220>
   <223> DNA fragment containing attR
<400> 14
   ctgcagtctg ttacaggtca ctaataccat ctaagtagtt gattcatagt gactgcatat 60
   gttgtgtttt acagtattat gtagtctgtt ttttatgcaa aatctaattt aatatattga 120
   tatttatatc attttacgtt tctcgttcag cttttttata ctaacttgag cgtctagaaa 180
   gctt 184
<210> 15
   <211> 41
   <212> DNA
   <213> Artificial
<220>
   <223> primer
<400> 15
   atgccactgc agtctgttac-aggtcactaa taccatctaa g 41
<210> 16
   <211> 46
   <212> DNA
   <213> Artificial
<220>
   <223> primer
<400> 16
   accgttaagc tttctagacg ctcaagttag tataaaaaag ctgaac 46
<210> 17
   <211> 38
   <212> DNA
   <213> Artificial
<220>
   <223> primer
<400> 17
   ttcttagacg tcaggtggca cttttcgggg aaatgtgc 38
<210> 18
   <211> 37
   <212> DNA
   <213> Artificial
<220>
   <223> primer
<400> 18
   taacagagat ctcgcgcaga aaaaaaggat ctcaaga 37
<210> 19
   <211> 46
   <212> DNA
   <213> Artificial
<220>
   <223> primer
<400> 19
   aacagagatc taagcttaga tcctttgcct ggcggcagta gcgcgg 46
<210> 20
   <211> 35
   <212> DNA
   <213> Artificial
<220>
   <223> primer
<400> 20
   ataaactgca gcaaaaagag tttgtagaaa cgcaa 35
<210> 21
   <211> 1388
   <212> DNA
   <213> Artificial
<220>
   <223> DNA fragment containing Tc gene and ter_thrL
<400> 21
   gaattctcat gtttgacagc ttatcatcga taagctttaa tgcggtagtt tatcacagtt     60
   aaattgctaa cgcagtcagg caccgtgtat gaaatctaac aatgcgctca tcgtcatcct    120
   cggcaccgtc accctggatg ctgtaggcat aggcttggtt atgccggtac tgccgggcct    180
   cttgcgggat atcgtccatt ccgacagcat cgccagtcac tatggcgtgc tgctagcgct    240
   atatgcgttg atgcaatttc tatgcgcacc cgttctcgga gcactgtccg accgctttgg    300
   ccgccgccca gtcctgctcg cttcgctact tggagccact atcgactacg cgatcatggc    360
   gaccacaccc gtcctgtgga tcctctacgc cggacgcatc gtggccggca tcaccggcgc    420
   cacaggtgcg gttgctggcg cctatatcgc cgacatcacc gatggggaag atcgggctcg    480
   ccacttcggg ctcatgagcg cttgtttcgg cgtgggtatg gtggcaggcc ccgtggccgg    540
   gggactgttg ggcgccatct ccttgcatgc accattcctt gcggcggcgg tgctcaacgg    600
   cctcaaccta ctactgggct gcttcctaat gcaggagtcg cataagggag agcgtcgacc    660
   gatgcccttg agagccttca acccagtcag ctccttccgg tgggcgcggg gcatgactat    720
   cgtcgccgca cttatgactg tcttctttat catgcaactc gtaggacagg tgccggcagc    780
   gctctgggtc attttcggcg aggaccgctt tcgctggagc gcgacgatga tcggcctgtc    840
   gcttgcggta ttcggaatct tgcacgccct cgctcaagcc ttcgtcactg gtcccgccac    900
   caaacgtttc ggcgagaagc aggccattat cgccggcatg gcggccgacg cgctgggcta    960
   cgtcttgctg gcgttcgcga cgcgaggctg gatggccttc cccattatga ttcttctcgc   1020
   ttccggcggc atcgggatgc ccgcgttgca ggccatgctg tccaggcagg tagatgacga   1080
   ccatcaggga cagcttcaag gatcgctcgc ggctcttacc agcctaactt cgatcactgg   1140
   accgctgatc gtcacggcga tttatgccgc ctcggcgagc acatggaacg ggttggcatg   1200
   gattgtaggc gccgccctat accttgtctg cctccccgcg ttgcgtcgcg gtgcatggag   1260
   ccgggccacc tcgacctgaa tggaagccgg cggcacctcg ctaacggatt caccactcca   1320
   actagaaagc ttaacacaga aaaaagcccg cacctgacag tgcgggcttt ttttttcgac   1380
   cactgcag                                                             1388
<210> 22
   <211> 36
   <212> DNA
   <213> Artificial
<220>
   <223> primer
<400> 22
   agtaattcta gaaagcttaa cacagaaaaa agcccg 36
<210> 23
   <211> 43
   <212> DNA
   <213> Artificial
<220>
   <223> primer
<400> 23
   ctagtaggat ccctgcagtg gtcgaaaaaa aaagcccgca ctg 43
<210> 24
   <211> 1162
   <212> DNA
   <213> Artificial
<220>
   <223> DNA fragment containing Pa2 promoter
<400> 24
   agatctccgg ataagtagac agcctgataa gtcgcacgaa aaacaggtat tgacaacatg     60
   aagtaacatg cagtaagata caaatcgcta ggtaacacta gcagcgtcaa ccgggcgctc    120
   tagctagagc caagctagct tggccggatc cgagattttc aggagctaag gaagctaaaa    180
   tggagaaaaa aatcactgga tataccaccg ttgatatatc ccaatggcat cgtaaagaac    240
   attttgaggc atttcagtca gttgctcaat gtacctataa ccagaccgtt cagctggata    300
   ttacggcctt tttaaagacc gtaaagaaaa ataagcacaa gttttatccg gcctttattc    360
   acattcttgc ccgcctgatg aatgctcatc cggaattccg tatggcaatg aaagacggtg    420
   agctggtgat atgggatagt gttcaccctt gttacaccgt tttccatgag caaactgaaa    480
   cgttttcatc gctctggagt gaataccacg acgatttccg gcagtttcta cacatatatt    540
   cgcaagatgt ggcgtgttac ggtgaaaacc tggcctattt ccctaaaggg tttattgaga    600
   atatgttttt cgtctcagcc aatccctggg tgagtttcac cagttttgat ttaaacgtgg    660
   ccaatatgga caacttcttc gcccccgttt tcaccatggg caaatattat acgcaaggcg    720
   acaaggtgct gatgccgctg gcgattcagg ttcatcatgc cgtctgtgat ggcttccatg    780
   tcggcagaat gcttaatgaa ttacaacagt actgcgatga gtggcagggc ggggcgtaat    840
   ttttttaagg cagttattgg tgcccttaaa cgcctggtgc tacgcctgaa taagtgataa    900
   taagcggatg aatggcagaa attcgtcgaa gcttaacaca gaaaaaagcc cgcacctgac    960
   agtgcgggct ttttttttcg accactgcag tctgttacag gtcactaata ccatctaagt   1020
   agttgattca tagtgactgc atatgttgtg ttttacagta ttatgtagtc tgttttttat    1080
   gcaaaatcta atttaatata ttgatattta tatcatttta cgtttctcgt tcagcttttt   1140
   tatactaact tgagcgtcta ga                                            1162
<210> 25
   <211> 37
   <212> DNA
   <213> Artificial sequence
<220>
   <223> primer
<400> 25
   atcgaggtac cagatctccg gataagtaga cagcctg 37
<210> 26
   <211> 32
   <212> DNA
   <213> Artificial sequence
<220>
   <223> primer
<400> 26
   gaaggtctag agcgcccggt tgacgctgct ag 32
<210> 27
   <211> 27
   <212> DNA
   <213> Artificial sequence
<220>
   <223> primer
<400> 27
   ctaatatcga tgaagattct tgctcaa 27
<210> 28
   <211> 34
   <212> DNA
   <213> Artificial sequence
<220>
   <223> primer
<400> 28
   gcgttgaatt ccatacaacc tccttagtac atgc 34
<210> 29
   <211> 34
   <212> DNA
   <213> Artificial sequence
<220>
   <223> primer
<400> 29
   gtactagaat tcgtgtaatt gcggagactt tgcg 34
<210> 30
   <211> 41
   <212> DNA
   <213> Artificial
<220>
   <223> primer
<400> 30
   aatagcctgc agttatttga tttcaatttt gtcccactcc c 41
<210> 31
   <211> 180
   <212> DNA
   <213> Artificial
<220>
   <223> DNA fragment containing PL-tac promoter
<400> 31
   ttagatctct cacctaccaa acaatgcccc cctgcaaaaa ataaattcat aaaaaacata 60
   cagataacca tctgcggtga taaattatct ctggcggtgt tgacaattaa tcatcggctc 120
   gtataatgtg tggaattgtg agcgtgctta agcttctgaa cctaagagga tgctatggga 180
<210> 32
   <211> 60
   <212> DNA
   <213> Artificial
<220>
   <223> primer
<400> 32
   ggcatgaaat ttcgattatt aaagtgatgg tagtcacgct caagttagta taaaaaagct 60
<210> 33
   <211> 40
   <212> DNA
   <213> Artificial
<220>
   <223> primer
<400> 33
   ctagtaagat cttgaagcct gcttttttat actaagttgg 40
<210> 34
   <211> 55
   <212> DNA
   <213> Artificial
<220>
   <223> primer
<400> 34
   tcccatagca tcctcttagg ttcagaagct taagcacgct cacaattcca cacat 55
<210> 35
   <211> 19
   <212> DNA
   <213> Artificial
<220>
   <223> primer
<400> 35
   agctacctct ctctgattc 19
<210> 36
   <211> 19
   <212> DNA
   <213> Artificial
<220>
   <223> primer
<400> 36
   tgtttcccat agcatcctc 19
<210> 37
   <211> 60
   <212> DNA
   <213> Artificial
<220>
   <223> primer
<400> 37
   cacaacacta aacctataag ttggggaaat acaatgtgaa gcctgctttt ttatactaag 60
<210> 38
   <211> 60
   <212> DNA
   <213> Artificial
<220>
   <223> primer
<400> 38
   gccgatgggc gccatttttc actgcggcaa gaattacgct caagttagta taaaaaagct 60
<210> 39
   <211> 22
   <212> DNA
   <213> Artificial
<220>
   <223> primer
<400> 39
   tcctggcatt gattcagcct gt 22
<210> 40
   <211> 21
   <212> DNA
   <213> Artificial
<220>
   <223> primer
<400> 40
   ccagcagcat gagagcgatg a 21